# EUROPEAN PATENT SPECIFICATION

(11) **EP 4 061 351 B1**
(45) Date of publication and mention of the grant of the patent: **16.09.2026**
(21) Application number: 20889265.3
(22) Date of filing: 17.11.2020
(51) Int. Cl.: A61K 31/167, C07C 233/64, C07C 233/75, A61P 35/00, C07C 235/64

(54) **DUAL ANDROGEN RECEPTOR/AKR1C3 INHIBITORS**
DOPPELTE ANDROGENREZEPTOR/AKR1C3-INHIBITOREN
INHIBITEURS DOUBLES DE RÉCEPTEUR DES ANDROGÈNES/AKR1C3

(30) Priority: 18.11.2019 US 201962937136 P
(43) Date of publication of application: 28.09.2022
(73) Proprietor: The Regents of the University of California, Oakland, CA 94607-5200 (US); Ohio State Innovation Foundation, Columbus, OH 43201 (US); The United States Government as represented by the Department of Veterans Affairs, Washington, DC 20420 (US)
(72) Inventor: GAO, Allen C., Oakland, California 94607-5200 (US); LI, Pui-Kai, Columbus, Ohio 43201 (US); XING, Enming, Columbus, Ohio 43201 (US); CHENG, Xiaolin, Columbus, Ohio 43201 (US); KONG, Xiaotian, Columbus, Ohio 43201 (US); LOU, Wei, Oakland, California 94607-5200 (US)
(74) Representative: J A Kemp LLP
(86) International application number: PCT/US2020/060907
(87) International publication number: WO 2021/101903

(56) References cited:
- WO-A1-2015/065919
- WO-A1-2015/065919
- US-A1- 2006 035 979
- US-A1- 2014 294 957
- US-A1- 2019 060 330
- US-B2- 8 097 759
- US-B2- 8 097 759
- DATABASE Pubchem Compound 17 September 2005 (2005-09-17), "2-hydroxy-N-phenyl-3-(phenylmethyl)-", XP055828106, retrieved from NCBI Database accession no. 5004151

## Description

### CROSS-REFERENCES TO RELATED APPLICATIONS

The present application claims priority to U.S. Provisional Pat. Appl. No. 62/937,136, filed on November 18, 2019.

### BACKGROUND OF THE INVENTION

Next-generation antiandrogens such as enzalutamide, apalutamide, and darolutamide, and androgen synthesis inhibitors such as abiraterone have been approved for treatment of castration resistant prostate cancer (CRPC) patients both before and after chemotherapy. These therapies, although effective initially, rapidly result in development of resistance to the agents resulting in progression of disease. Urgent ongoing work is being conducted to unravel potential resistance mechanisms in order to devise ways of targeting resistance pathways that perpetuate disease progression during effective AR blockade. Considerable evidence from both clinical and experimental studies demonstrates that androgen receptor (AR) and its variant 7 (AR-V7) play vital roles in promoting CRPC progression during androgen deprivation therapy and induction of resistance to enzalutamide and abiraterone therapy. Recent studies also suggest that AKR1C3 activation and enhanced synthesis of intracrine androgens contribute to enzalutamide and abiraterone resistance. AKR1C3 is overexpressed and intracrine androgens are elevated in enzalutamide/abiraterone resistant prostate cancer cells. WO 2015/065919 A1 describes the treatment of metastatic prostate cancer.

### BRIEF SUMMARY OF THE INVENTION

Provided herein are compounds according to Formula I: 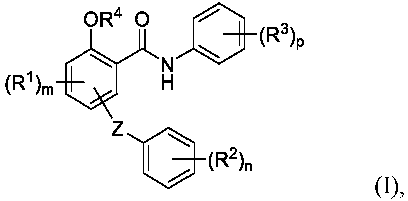 or a pharmaceutically acceptable salt thereof, wherein:
Z is selected from the group consisting of -CH₂-, -C(O)-, -O-, -S-, and -MR^{a}-;
Z is in the para position with respect to -OR⁴ or the ortho position with respect to -C(O)NH-;
subscript m is 0, 1, 2, or 3;
each R¹ is independently selected from the group consisting of -OH, C₁₋₈ alkyl, C₁₋₈ alkoxy, and halogen;
subscript n is 0, 1, 2, 3, 4, or 5;
each R² is independently selected from the group consisting of -OH, halogen, -NO₂, -CN, C₁₋₈ alkyl, C₁₋₈ alkoxy, C₁₋₈ haloalkyl, C₂₋₈ alkenyl, C₂₋₈ alkynyl, and -N(R^{a})₂;
subscript p is 2, 3, 4, 5, or 1;
each R³ is independently selected from the group consisting of C₁₋₈ haloalkyl, -OH, halogen, -NO₂, -CN, C₁₋₈ alkyl, C₁₋₈ alkoxy, C₂₋₈ alkenyl, C₂₋₈ alkynyl, and -N(R^{a})₂;
R⁴ is selected from the group consisting of H, α-aminoacyl; -S(O)₂N(R^{a})₂, -C(O)N(R^{a})₂, and -C(O)R^{b};
each R^{a} is independently selected from the group consisting of H and C₁₋₈ alkyl;
R^{b} is C₁₋₈ alkyl;
provided that:
   if Z is -CH₂- in the para position with respect to -OR⁴, subscript m and subscript n are 0, and R⁴ is H, then at least one R³ is selected from the group consisting of C₁₋₈ haloalkyl, halogen, -NO₂, C₂₋₈ alkyl, C₁₋₈ alkoxy, C₂₋₈ alkenyl, C₂₋₈ alkynyl, and -N(R^{a})₂;
   if Z is -CH₂- in the para position with respect to -OR⁴ and the compound is a 2,3,4-trihydroxy-benzamide, then at least one R² or at least one R³ is selected from the group consisting of -OH,-NO₂, -CN, C₁₋₈ alkoxy, C₁₋₈ haloalkyl, C₂₋₈ alkenyl, C₂₋₈ alkynyl, and -N(R^{a})₂;
   if Z is -CH₂- in the para position with respect to -OR⁴ and the compound is a 5-alkyl-6-hydroxy-benzamide, then at least one R² or at least one R³ is selected from the group consisting of -OH,-NO₂, -CN, C₁₋₈ alkyl, C₁₋₈ alkoxy, C₁₋₈ haloalkyl, C₂₋₈ alkenyl, C₂₋₈ alkynyl, and -N(R^{a})₂;
   if Z is -C(O)- in the para position with respect to -OR⁴ and the compound is a 2-hydroxy-benzamide, a 2,5-dialkyl-6-hydroxy-benzamide, or a 5-alkyl-6-hydroxy-benzamide, then (i) at least one R² or at least one R³ is selected from the group consisting of - OH, C₁₋₈ alkoxy, C₁₋₈ haloalkyl, C₂₋₈ alkenyl, C₂₋₈ alkynyl, and -N(R^{a})₂, and (ii) at least one R³ is other than -CF₃ or -NO₂ when subscript n is 1 and R² is 4-chloro or 4-(n-butyl); and
   if Z is -S- in the para position with respect to -OR⁴ and the compound is a 2,5-dialkyl-6-hydroxy-benzamide or a 5-alkyl-6-hydroxy-benzamide, then at least one R² or at least one R³ is selected from the group consisting of -OH, C₂₋₈ alkyl, C₂₋₈ alkoxy, C₂₋₈ alkenyl, C₂₋₈ alkynyl, and -N(R^{a})₂.

Also provided herein are pharmaceutical compositions containing one or more compounds as described herein and one or more pharmaceutically acceptable excipients.

Also provided herein is a compound as described herein or a pharmaceutical composition described herein for use as a medicament.

Also provided herein is a compound or composition described herein for use in methods for treating a hormone-mediated disease or condition such as cancer, e.g., castration-resistant prostate cancer. The methods include administering a therapeutically effective amount of the compound or composition as described herein to a subject in need thereof, thereby treating the hormone-mediated disease or condition.

### BRIEF DESCRIPTION OF THE DRAWINGS

FIG. 1 shows a Western blot analysis for AR-FL, AR-Vs, and AKR1C3 protein expression after CWR22rv1 cells were treated with compound **1** and compound **2.**
FIG. 2 shows a plot of enzymatic activity of LNCaP-AKR1C3 cells treated with either compound **1** or compound **2** over 48 hrs, demonstrating that the compounds inhibit AKR1C3 activity.
FIG. 3 shows a plot of enzymatic activity in LNCaP-AKR1C3 cells treated with increasing doses of compound **1** over 48 hrs, demonstrating that AKR1C3 is inhibited in dose-dependent fashion.
FIG. 4 shows total cell numbers measured after treating C4-2B MDVR cells with either compound **1** or compound **2,** demonstrating that the compounds inhibit cell growth.
FIG. 5 shows total cell numbers measured after treating C4-2B MDVR cells with increasing doses of compound **1** over 48 hrs, demonstrating that cell growth is inhibited in dose-dependent fashion.
FIG. 6 shows total cell numbers measured after treating enzalutamide (Enza)-resistant MDVR cells, abiraterone (Abi)-resistant AbiR cells, apalutamide (Apal)-resistant ApalR cells, and darolutamide (Daro)-resistant DaroR cells with the respective anti-androgens Enza (20 µM), Abi (10 µM), Apal (20 µM), Daro (20 µM) or compound **1** (5 µM) for 48 hrs.

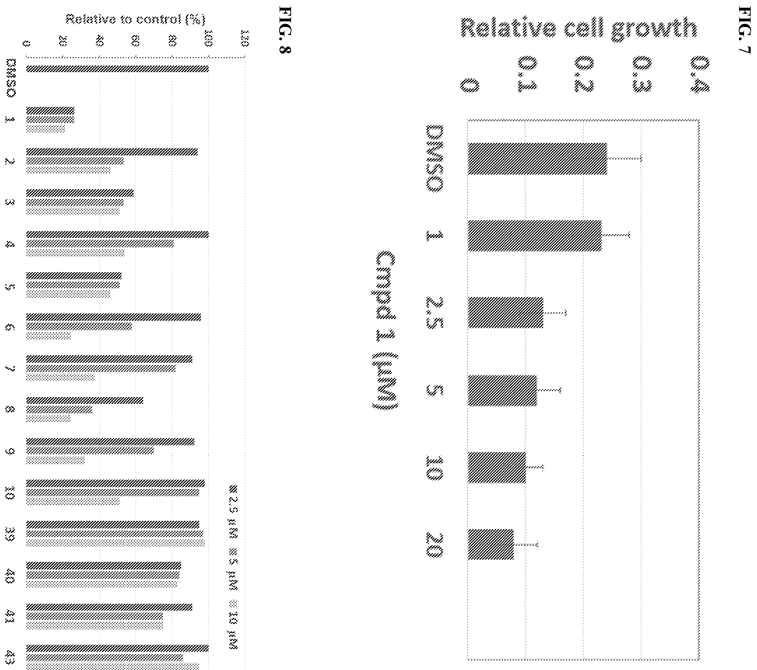

FIG. 7 shows total cell numbers measured after treating MCF-7 breast cancer cells with increasing doses of compound **1** over 48 hrs, demonstrating that cell growth is inhibited in dose dependent fashion.
FIG. 8 shows total cell numbers measured after treating C4-2B AKR1C3 cells with different compounds over 48 hrs.
FIG. 9A shows the volume of VCaP tumors in mice treated with compound **1** and a vehicle control.
FIG. 9B shows the body weight of mice treated with compound **1** and a vehicle control in the VCaP model.
FIG. 9C shows the levels of prostate specific antigen (PSA) in mice treated with compound **1** and a vehicle control in the VCaP model. Taken together, FIGS. 9A-9C show that compound **1** inhibits VCaP tumor growth.
FIG. 10A shows the volume of LuCaP35CR tumors in mice treated with compound **1** and a vehicle control.
FIG. 10B shows the body weight of mice treated with compound **1** and a vehicle control in the LuCaP35CR model.
FIG. 10C shows the levels of prostate specific antigen (PSA) in mice treated with compound **1** and a vehicle control in the LuCaP35CR model.
FIG. 10D shows intratumoral testosterone levels in mice treated with compound **1** and a vehicle control in the LuCaP35CR model. Taken together, FIGS. 10A-10D show that compound **1** inhibits LuCaP35CR tumor growth.
FIG. 11 shows that compound **1** inhibits the conversion from androstenedione to testosterone in LuCaP35CR tumors *ex vivo.*
FIG. 12 shows that compound **1** and compound **5** inhibit AR nuclear translocation in LNCaP cells. LNCaP cells cultured in charcoal stripped FBS conditions were treated with DHT and the compounds as indicated. AR expression were visualized by immunofluorescent staining.
FIG. 13A shows an RNAseq heatmap demonstrating that compound **1** and compound **5** inhibit AR activity signature genes in C4-2B MDVR cells.
FIG. 13B shows an RNAseq heatmap demonstrating that compound **1** and compound 5 inhibit AR-V7 activity signature genes in C4-2B MDVR cells.
FIG. 14 shows an RNAseq heatmap demonstrating that compounds **1, 2, 5,** and **8** inhibit genes associated with cancer cell proliferation and survival in C4-2B MDVR cells.

### DETAILED DESCRIPTION OF THE INVENTION

The present invention is based, in part, on the recognition that inhibition of both AR/ARv7 and AKR1C3 would be an ideal strategy for treating hormone-related cancers, such as advanced prostate cancer, and overcoming resistance to current therapies. Overexpression of AKR1C3 confers resistance to enzalutamide/abiraterone, while down regulation of AKR1C3 sensitizes cells to enzalutamide/abiraterone treatment. Importantly, overexpression of AKR1C3 has been demonstrated in clinical metastatic prostate cancer. As described herein, a number of new benzamide compounds have been surprisingly found to function as dual inhibitors of AR and AKR1C3. The benzamides inhibit both expression and activity of AR/ARv7 and AKR1C3 in prostate cancer cells, as well as the growth of resistant prostate cancer cells. Furthermore, the compounds inhibit enzalutamide-resistant C4-2B MDVR cell growth, abiraterone-resistant cell growth, apalutamide-resistant cell growth, and darolutamide-resistant cell growth.

### I. Definitions

As used herein, the term "alkyl," by itself or as part of another substituent, refers to a straight or branched, saturated, aliphatic radical having the number of carbon atoms indicated. Alkyl can include any number of carbons, such as C₁₋₂, C₁₋₃, C₁₋₄, C₁₋₅, C₁₋₆, C₁₋₇, C₁₋₈, C₁₋₉, C₁₋₁₀, C₂₋₃, C₂₋₄, C₂₋₅, C₂₋₆, C₃₋₄, C₃₋₅, C₃₋₆, C₄₋₅, C₄₋₆, and C₅₋₆. For example, C₁₋₆ alkyl includes, but is not limited to, methyl, ethyl, propyl, isopropyl, butyl, isobutyl, sec-butyl, tert-butyl, pentyl, isopentyl, hexyl, etc. Alkyl can also refer to alkyl groups having up to 20 carbon atoms, such as, but not limited to heptyl, octyl, nonyl, decyl, etc. Alkyl groups can be substituted or unsubstituted. Unless otherwise specified, "substituted alkyl" groups may be substituted with one or more groups selected from halo, hydroxy, amino, alkylamino, amido, acyl, nitro, cyano, and alkoxy.

As used herein, the term "alkoxy," by itself or as part of another substituent, refers to a group having the formula -OR, wherein R is alkyl as described above.

As used herein, the term "alkenyl," by itself or as part of another substituent, refers to a straight chain or branched hydrocarbon having at least 2 carbon atoms and at least one double bond. Alkenyl can include any number of carbons, such as C₂, C₂₋₃, C₂₋₄, C₂₋₅, C₂₋₆, C₂₋₇, C₂₋₈, C₂₋₉, C₂₋₁₀, C₃, C₃₋₄, C₃₋₅, C₃₋₆, C₄, C₄₋₅, C₄₋₆, C₅, C₅₋₆, and C₆. Alkenyl groups can have any suitable number of double bonds, including, but not limited to, 1, 2, 3, 4, 5 or more. Examples of alkenyl groups include, but are not limited to, vinyl (ethenyl), propenyl, isopropenyl, 1-butenyl, 2-butenyl, isobutenyl, butadienyl, 1-pentenyl, 2-pentenyl, isopentenyl, 1,3-pentadienyl, 1,4-pentadienyl, 1-hexenyl, 2-hexenyl, 3-hexenyl, 1,3-hexadienyl, 1,4-hexadienyl, 1,5-hexadienyl, 2,4-hexadienyl, or 1,3,5-hexatrienyl. Alkenyl groups can be substituted or unsubstituted. Unless otherwise specified, "substituted alkenyl" groups may be substituted with one or more moieties selected from halo, hydroxy, amino, alkylamino, alkoxy, haloalkyl, carboxy, amido, nitro, oxo, and cyano.

As used herein, the term "alkynyl," by itself or as part of another substituent, refers to either a straight chain or branched hydrocarbon having at least 2 carbon atoms and at least one triple bond. Alkynyl can include any number of carbons, such as C₂, C₂₋₃, C₂₋₄, C₂₋₅, C₂₋₆, C₂₋₇, C₂₋₈, C₂₋₉, C₂₋₁₀, C₃, C₃₋₄, C₃₋₅, C₃₋₆, C₄, C₄₋₅, C₄₋₆, C₅, C₅₋₆, and C₆. Examples of alkynyl groups include, but are not limited to, acetylenyl, propynyl, 1-butynyl, 2-butynyl, isobutynyl, sec-butynyl, butadiynyl, 1-pentynyl, 2-pentynyl, isopentynyl, 1,3-pentadiynyl, 1,4-pentadiynyl, 1-hexynyl, 2-hexynyl, 3-hexynyl, 1,3-hexadiynyl, 1,4-hexadiynyl, 1,5-hexadiynyl, 2,4-hexadiynyl, or 1,3,5-hexatriynyl. Alkynyl groups can be substituted or unsubstituted. Unless otherwise specified, "substituted alkynyl" groups may be substituted with one or more moieties selected from halo, hydroxy, amino, alkylamino, alkoxy, haloalkyl, carboxy, amido, nitro, oxo, and cyano.

As used herein, the terms "halo" and "halogen" refer to fluorine, chlorine, bromine and iodine.

As used herein, the term "haloalkyl," by itself or as part of another substituent, refers to an alkyl group where some or all of the hydrogen atoms are replaced with halogen atoms. As for alkyl groups, haloalkyl groups can have any suitable number of carbon atoms, such as C₁₋₆. For example, haloalkyl includes trifluoromethyl, fluoromethyl, *etc.* In some instances, the term "perfluoro" can be used to define a compound or radical where all the hydrogens are replaced with fluorine. For example, perfluoromethyl refers to 1,1,1-trifluoromethyl.

As used herein, the term "hydroxy" refers to the moiety -OH.

As used herein, the term "oxo" refers to an oxygen atom that is double-bonded to a compound (*i.e.,* O=).

As used herein, the term "amino" refers to a moiety -NR₂, wherein each R group is H or alkyl. An amino moiety can be ionized to form the corresponding ammonium cation. "Alkylamino" refers to an amino moiety wherein at least one of the R groups is alkyl.

As used herein, the term "α-aminoacyl" refers to a moiety -C(O)CNR'R", wherein R' and R" are independently hydrogen, alkyl, alkynyl, aryl, heteroaryl, cycloaclkyl, or heterocyclyl, each of which is optionally substituted with one or more substituents selected from halo, hydroxy, amino, alkylamino, amido, acyl, nitro, cyano, and alkoxy.

As used herein, the term "aryl," by itself or as part of another substituent, refers to an aromatic ring system having any suitable number of carbon ring atoms and any suitable number of rings. Aryl groups can include any suitable number of carbon ring atoms, such as C₆, C₇, C₈, C₉, C₁₀, C₁₁, C₁₂, C₁₃, C₁₄, C₁₅ or C₁₆, as well as C₆₋₁₀, C₆₋₁₂, or C₆₋₁₄ . Aryl groups can be monocyclic, fused to form bicyclic (*e*.*g*., benzocyclohexyl) or tricyclic groups, or linked by a bond to form a biaryl group. Representative aryl groups include phenyl, naphthyl and biphenyl. Other aryl groups include benzyl, having a methylene linking group. Some aryl groups have from 6 to 12 ring members, such as phenyl, naphthyl or biphenyl. Other aryl groups have from 6 to 10 ring members, such as phenyl or naphthyl. Some other aryl groups have 6 ring members, such as phenyl. Aryl groups can be substituted or unsubstituted. Unless otherwise specified, "substituted aryl" groups can be substituted with one or more groups selected from halo, hydroxy, amino, alkylamino, amido, acyl, nitro, cyano, and alkoxy.

As used herein, the term "heteroaryl," by itself or as part of another substituent, refers to a monocyclic or fused bicyclic or tricyclic aromatic ring assembly containing 5 to 16 ring atoms, where from 1 to 5 of the ring atoms are a heteroatom such as N, O or S. Additional heteroatoms can also be useful, including, but not limited to, B, Al, Si and P. The heteroatoms can be oxidized to form moieties such as, but not limited to, -S(O)- and -S(O)₂-. Heteroaryl groups can include any number of ring atoms, such as C₅₋₆, C₃₋₈, C₄₋₈, C₅₋₈, C₆₋₈, C₃₋₉, C₃₋₁₀, C₃₋₁₁, or C₃₋₁₂, wherein at least one of the carbon atoms is replaced by a heteroatom. Any suitable number of heteroatoms can be included in the heteroaryl groups, such as 1, 2, 3, 4; or 5, or 1 to 2, 1 to 3, 1 to 4, 1 to 5, 2 to 3, 2 to 4, 2 to 5, 3 to 4, or 3 to 5. For example, heteroaryl groups can be C₅₋₈ heteroaryl, wherein 1 to 4 carbon ring atoms are replaced with heteroatoms; or C₅₋₈ heteroaryl, wherein 1 to 3 carbon ring atoms are replaced with heteroatoms; or C₅₋₆ heteroaryl, wherein 1 to 4 carbon ring atoms are replaced with heteroatoms; or C₅₋₆ heteroaryl, wherein 1 to 3 carbon ring atoms are replaced with heteroatoms. The heteroaryl group can include groups such as pyrrole, pyridine, imidazole, pyrazole, triazole, tetrazole, pyrazine, pyrimidine, pyridazine, triazine (1,2,3-, 1,2,4- and 1,3,5-isomers), thiophene, furan, thiazole, isothiazole, oxazole, and isoxazole. The heteroaryl groups can also be fused to aromatic ring systems, such as a phenyl ring, to form members including, but not limited to, benzopyrroles such as indole and isoindole, benzopyridines such as quinoline and isoquinoline, benzopyrazine (quinoxaline), benzopyrimidine (quinazoline), benzopyridazines such as phthalazine and cinnoline, benzothiophene, and benzofuran. Other heteroaryl groups include heteroaryl rings linked by a bond, such as bipyridine. Heteroaryl groups can be substituted or unsubstituted. Unless otherwise specified, "substituted heteroaryl" groups can be substituted with one or more groups selected from halo, hydroxy, amino, alkylamino, amido, acyl, nitro, cyano, and alkoxy.

The heteroaryl groups can be linked via any position on the ring. For example, pyrrole includes 1-, 2- and 3-pyrrole, pyridine includes 2-, 3- and 4-pyridine, imidazole includes 1-, 2-, 4- and 5-imidazole, pyrazole includes 1-, 3-, 4- and 5-pyrazole, triazole includes 1-, 4- and 5-triazole, tetrazole includes 1- and 5-tetrazole, pyrimidine includes 2-, 4-, 5- and 6- pyrimidine, pyridazine includes 3- and 4-pyridazine, 1,2,3-triazine includes 4- and 5-triazine, 1,2,4-triazine includes 3-, 5- and 6-triazine, 1,3,5-triazine includes 2-triazine, thiophene includes 2- and 3-thiophene, furan includes 2- and 3-furan, thiazole includes 2-, 4- and 5-thiazole, isothiazole includes 3-, 4- and 5-isothiazole, oxazole includes 2-, 4- and 5-oxazole, isoxazole includes 3-, 4- and 5-isoxazole, indole includes 1-, 2- and 3-indole, isoindole includes 1- and 2-isoindole, quinoline includes 2-, 3- and 4-quinoline, isoquinoline includes 1-, 3- and 4-isoquinoline, quinazoline includes 2- and 4-quinazoline, cinnoline includes 3- and 4-cinnoline, benzothiophene includes 2- and 3-benzothiophene, and benzofuran includes 2- and 3-benzofuran.

Some heteroaryl groups include those having from 5 to 10 ring members and from 1 to 3 ring atoms including N, O or S, such as pyrrole, pyridine, imidazole, pyrazole, triazole, pyrazine, pyrimidine, pyridazine, triazine (1,2,3-, 1,2,4- and 1,3,5-isomers), thiophene, furan, thiazole, isothiazole, oxazole, isoxazole, indole, isoindole, quinoline, isoquinoline, quinoxaline, quinazoline, phthalazine, cinnoline, benzothiophene, and benzofuran. Other heteroaryl groups include those having from 5 to 8 ring members and from 1 to 3 heteroatoms, such as pyrrole, pyridine, imidazole, pyrazole, triazole, pyrazine, pyrimidine, pyridazine, triazine (1,2,3-, 1,2,4- and 1,3,5-isomers), thiophene, furan, thiazole, isothiazole, oxazole, and isoxazole. Some other heteroaryl groups include those having from 9 to 12 ring members and from 1 to 3 heteroatoms, such as indole, isoindole, quinoline, isoquinoline, quinoxaline, quinazoline, phthalazine, cinnoline, benzothiophene, benzofuran and bipyridine. Still other heteroaryl groups include those having from 5 to 6 ring members and from 1 to 2 ring atoms including N, O or S, such as pyrrole, pyridine, imidazole, pyrazole, pyrazine, pyrimidine, pyridazine, thiophene, furan, thiazole, isothiazole, oxazole, and isoxazole.

Some heteroaryl groups include from 5 to 10 ring members and only nitrogen heteroatoms, such as pyrrole, pyridine, imidazole, pyrazole, triazole, pyrazine, pyrimidine, pyridazine, triazine (1,2,3-, 1,2,4- and 1,3,5-isomers), indole, isoindole, quinoline, isoquinoline, quinoxaline, quinazoline, phthalazine, and cinnoline. Other heteroaryl groups include from 5 to 10 ring members and only oxygen heteroatoms, such as furan and benzofuran. Some other heteroaryl groups include from 5 to 10 ring members and only sulfur heteroatoms, such as thiophene and benzothiophene. Still other heteroaryl groups include from 5 to 10 ring members and at least two heteroatoms, such as imidazole, pyrazole, triazole, pyrazine, pyrimidine, pyridazine, triazine (1,2,3-, 1,2,4- and 1,3,5-isomers), thiazole, isothiazole, oxazole, isoxazole, quinoxaline, quinazoline, phthalazine, and cinnoline.

As used herein, the term "cycloalkyl," by itself or as part of another substituent, refers to a saturated or partially unsaturated, monocyclic, fused bicyclic or bridged polycyclic ring assembly containing from 3 to 12 ring atoms, or the number of atoms indicated. Cycloalkyl can include any number of carbons, such as C₃₋₆, C₄₋₆, C₅₋₆, C₃₋₈, C₄₋₈, C₅₋₈, C₆₋₈, C₃₋₉, C₃₋₁₀, C₃₋₁₁, and C₃₋₁₂. Saturated monocyclic cycloalkyl rings include, for example, cyclopropyl, cyclobutyl, cyclopentyl, cyclohexyl, and cyclooctyl. Saturated bicyclic and polycyclic cycloalkyl rings include, for example, norbornane, [2.2.2] bicyclooctane, decahydronaphthalene and adamantane. Cycloalkyl groups can also be partially unsaturated, having one or more double or triple bonds in the ring. Representative cycloalkyl groups that are partially unsaturated include, but are not limited to, cyclobutene, cyclopentene, cyclohexene, cyclohexadiene (1,3- and 1,4-isomers), cycloheptene, cycloheptadiene, cyclooctene, cyclooctadiene (1,3-, 1,4- and 1,5-isomers), norbornene, and norbornadiene. When cycloalkyl is a saturated monocyclic C₃₋₈ cycloalkyl, exemplary groups include, but are not limited to cyclopropyl, cyclobutyl, cyclopentyl, cyclohexyl, cycloheptyl and cyclooctyl. When cycloalkyl is a saturated monocyclic C₃₋₆ cycloalkyl, exemplary groups include, but are not limited to cyclopropyl, cyclobutyl, cyclopentyl, and cyclohexyl. Cycloalkyl groups can be substituted or unsubstituted. Unless otherwise specified, "substituted cycloalkyl" groups can be substituted with one or more groups selected from halo, hydroxy, amino, alkylamino, amido, acyl, nitro, cyano, and alkoxy.

As used herein the term "heterocyclyl," by itself or as part of another substituent, refers to a saturated ring system having from 3 to 12 ring members and from 1 to 4 heteroatoms of N, O and S. Additional heteroatoms can also be useful, including, but not limited to, B, Al, Si and P. The heteroatoms can be oxidized to form moieties such as, but not limited to, -S(O)- and -S(O)₂-. Heterocyclyl groups can include any number of ring atoms, such as, C₃₋₆, C₄₋₆, C₅₋₆, C₃₋₈, C₄₋₈, C₅₋₈, C₆₋₈, C₃₋₉, C₃₋₁₀, C₃₋₁₁, or C₃₋₁₂, wherein at least one of the carbon atoms is replaced by a heteroatom. Any suitable number of carbon ring atoms can be replaced with heteroatoms in the heterocyclyl groups, such as 1, 2, 3, or 4, or 1 to 2, 1 to 3, 1 to 4, 2 to 3, 2 to 4, or 3 to 4. The heterocyclyl group can include groups such as aziridine, azetidine, pyrrolidine, piperidine, azepane, azocane, quinuclidine, pyrazolidine, imidazolidine, piperazine (1,2-, 1,3- and 1,4-isomers), oxirane, oxetane, tetrahydrofuran, oxane (tetrahydropyran), oxepane, thiirane, thietane, thiolane (tetrahydrothiophene), thiane (tetrahydrothiopyran), oxazolidine, isoxazolidine, thiazolidine, isothiazolidine, dioxolane, dithiolane, morpholine, thiomorpholine, dioxane, or dithiane. The heterocyclyl groups can also be fused to aromatic or non-aromatic ring systems to form members including, but not limited to, indoline. Heterocyclyl groups can be unsubstituted or substituted. Unless otherwise specified, "substituted heterocyclyl" groups can be substituted with one or more groups selected from halo, hydroxy, amino, oxo (=O), alkylamino, amido, acyl, nitro, cyano, and alkoxy.

The heterocyclyl groups can be linked via any position on the ring. For example, aziridine can be 1- or 2-aziridine, azetidine can be 1- or 2- azetidine, pyrrolidine can be 1-, 2- or 3-pyrrolidine, piperidine can be 1-, 2-, 3- or 4-piperidine, pyrazolidine can be 1-, 2-, 3-, or 4-pyrazolidine, imidazolidine can be 1-, 2-, 3- or 4-imidazolidine, piperazine can be 1-, 2-, 3- or 4-piperazine, tetrahydrofuran can be 1- or 2-tetrahydrofuran, oxazolidine can be 2-, 3-, 4- or 5-oxazolidine, isoxazolidine can be 2-, 3-, 4- or 5-isoxazolidine, thiazolidine can be 2-, 3-, 4- or 5-thiazolidine, isothiazolidine can be 2-, 3-, 4- or 5- isothiazolidine, and morpholine can be 2-, 3- or 4-morpholine.

When heterocyclyl includes 3 to 8 ring members and 1 to 3 heteroatoms, representative members include, but are not limited to, pyrrolidine, piperidine, tetrahydrofuran, oxane, tetrahydrothiophene, thiane, pyrazolidine, imidazolidine, piperazine, oxazolidine, isoxazolidine, thiazolidine, isothiazolidine, morpholine, thiomorpholine, dioxane and dithiane. Heterocyclyl can also form a ring having 5 to 6 ring members and 1 to 2 heteroatoms, with representative members including, but not limited to, pyrrolidine, piperidine, tetrahydrofuran, tetrahydrothiophene, pyrazolidine, imidazolidine, piperazine, oxazolidine, isoxazolidine, thiazolidine, isothiazolidine, and morpholine.

As used herein, the term "amido" refers to a moiety -NRC(O)R or -C(O)NR₂, wherein each R group is H or alkyl.

As used herein, the term "acyl" refers to the moiety -C(O)R, wherein each R group is alkyl.

As used herein, the term "nitro" refers to the moiety -NO₂.

As used herein, the term "cyano" refers to a carbon atom triple-bonded to a nitrogen atom (*i.e.,* the moiety -C≡N).

As used herein, the term "carboxy" refers to the moiety -C(O)OH.

As used herein, the term "salt" refers to an acid salt or base salt of an active agent such as an androgen receptor inhibitor or an AKR1C3 inhibitor. Acid salts of basic active agents include mineral acid salts (*e*.*g*., salts formed by using hydrochloric acid, hydrobromic acid, phosphoric acid, and the like), organic acid salts (*e*.*g*., salts formed using acetic acid, propionic acid, glutamic acid, citric acid, and the like), and quaternary ammonium salts (*e.g*., salts formed via reaction of an amine with methyl iodide, ethyl iodide, or the like). It is understood that the pharmaceutically acceptable salts are non-toxic.

Acidic active agents may be contacted with bases to provide base salts such as alkali and alkaline earth metal salts, such as sodium, lithium, potassium, calcium, magnesium, as well as ammonium salts, such as ammonium, trimethyl-ammonium, diethylammonium, and tris-(hydroxymethyl)-methyl-ammonium salts.

The neutral forms of the active agents can be regenerated by contacting the salt with a base or acid and isolating the parent compound in the conventional manner if desired. In some embodiments, the parent form of the compound may differ from various salt forms in certain physical properties, such as solubility in polar solvents, but otherwise the salts forms may be equivalent to the parent form of the compound.

By "pharmaceutically acceptable," it is meant that the excipient is compatible with the other ingredients of the formulation and is not deleterious to the recipient thereof. As used herein, the term "pharmaceutically acceptable excipient" refers to a substance that aids the administration of an active agent to a subject. Useful pharmaceutical excipients include, but are not limited to, binders, fillers, disintegrants, lubricants, glidants, coatings, sweeteners, flavors and colors.

As used herein, the terms "effective amount" and "therapeutically effective amount" refer to a dose of a compound such as androgen receptor inhibitor, an AKR1C3 inhibitor, or an antiandrogen that produces therapeutic effects for which it is administered. The exact dose will depend on the purpose of the treatment, and will be ascertainable by one skilled in the art using known techniques (*see, e.g.,* Lieberman, Pharmaceutical Dosage Forms (vols. 1-3, 1992); Lloyd, The Art, Science and Technology of Pharmaceutical Compounding (1999); Pickar, Dosage Calculations (1999); Goodman & Gilman's The Pharmacological Basis of Therapeutics, 11th Edition, 2006, Brunton, Ed., McGraw-Hill; and Remington: The Science and Practice of Pharmacy, 21st Edition, 2005, Hendrickson, Ed., Lippincott, Williams & Wilkins).

As used herein, the term "cancer" is intended to include any member of a class of diseases characterized by the uncontrolled growth of aberrant cells. The term includes all known cancers and neoplastic conditions, whether characterized as malignant, benign, recurrent, soft tissue, or solid, and cancers of all stages and grades including advanced, recurrent, pre- and post-metastatic cancers. Additionally, the term includes androgen-independent, castrate-resistant, castration recurrent, hormone-resistant, drug-resistant, and metastatic castrate-resistant cancers. Examples of different types of cancer include, but are not limited to, prostate cancer (*e.g.,* prostate adenocarcinoma); breast cancers (*e.g.,* triple-negative breast cancer, ductal carcinoma *in situ,* invasive ductal carcinoma, tubular carcinoma, medullary carcinoma, mucinous carcinoma, papillary carcinoma, cribriform carcinoma, invasive lobular carcinoma, inflammatory breast cancer, lobular carcinoma *in situ,* Paget's disease, Phyllodes tumors); gynecological cancers *(e.g.,* ovarian, cervical, uterine, vaginal, and vulvar cancers); lung cancers (*e*.*g*., non-small cell lung cancer, small cell lung cancer, mesothelioma, carcinoid tumors, lung adenocarcinoma); digestive and gastrointestinal cancers such as gastric cancer (*e*.*g*., stomach cancer), colorectal cancer, gastrointestinal stromal tumors (GIST), gastrointestinal carcinoid tumors, colon cancer, rectal cancer, anal cancer, bile duct cancer, small intestine cancer, and esophageal cancer; thyroid cancer; gallbladder cancer; liver cancer; pancreatic cancer; appendix cancer; renal cancer *(e.g.,* renal cell carcinoma); cancer of the central nervous system (*e.g.,* glioblastoma, neuroblastoma); skin cancer *(e.g.,* melanoma); bone and soft tissue sarcomas (*e.g.,* Ewing's sarcoma); lymphomas; choriocarcinomas; urinary cancers (*e*.*g*., urothelial bladder cancer); head and neck cancers; and bone marrow and blood cancers (*e*.*g*., chronic lymphocytic leukemia, lymphoma). As used herein, a "tumor" comprises one or more cancerous cells.

As used herein, the terms "antiandrogen" and "antiandrogen drug" refer to compounds that alter the androgen pathway by blocking the androgen receptors, competing for binding sites on the cell's surface, or affecting or mediating androgen production. Antiandrogens are useful for treating several diseases including, but not limited to, prostate cancer. Examples of antiandrogens include, but are not limited to, enzalutamide, abiraterone, bicalutamide, and darolutamide.

As used herein, the terms "about" and "around" indicate a close range around a numerical value when used to modify that specific value. If "X" were the value, for example, "about X" or "around X" would indicate a value from 0.9X to 1.1X, *e.g.,* a value from 0.95X to 1.05X, or a value from 0.98X to 1.02X, or a value from 0.99X to 1.01X. Any reference to "about X" or "around X" specifically indicates at least the values X, 0.9X, 0.91X, 0.92X, 0.93X, 0.94X, 0.95X, 0.96X, 0.97X, 0.98X, 0.99X, 1.01X, 1.02X, 1.03X, 1.04X, 1.05X, 1.06X, 1.07X, 1.08X, 1.09X, and 1.1X, and values within this range.

### II. AR/AKR1C3 Dual Inhibitors

Provided herein are compounds according to Formula **I:** 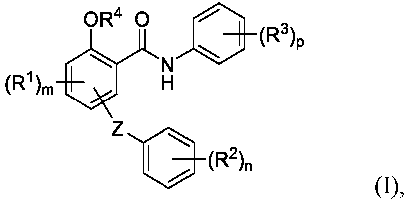 or a pharmaceutically acceptable salt thereof, wherein:
Z is selected from the group consisting of -CH₂-, -C(O)-, -O-, -S-, and -NR^{a}-;
Z is in the para position with respect to -OR⁴ or the ortho position with respect to -C(O)NH-;
subscript m is 0, 1, 2, or 3;
each R¹ is independently selected from the group consisting of -OH, C₁₋₈ alkyl, C₁₋₈ alkoxy, and halogen;
subscript n is 0, 1, 2, 3, 4, or 5;
each R² is independently selected from the group consisting of -OH, halogen, -NO₂, -CN, C₁₋₈ alkyl, C₁₋₈ alkoxy, C₁₋₈ haloalkyl, C₂₋₈ alkenyl, C₂₋₈ alkynyl, and -N(R^{a})₂;
subscript p is 2, 3, 4, 5, or 1;
each R³ is independently selected from the group consisting of C₁₋₈ haloalkyl, -OH, halogen, -NO₂, -CN, C₁₋₈ alkyl, C₁₋₈ alkoxy, C₂₋₈ alkenyl, C₂₋₈ alkynyl, and -N(R^{a})₂;
R⁴ is selected from the group consisting of H, α-aminoacyl; -S(O)₂N(R^{a})₂, -C(O)N(R^{a})₂, and -C(O)R^{b};
each R^{a} is independently selected from the group consisting of H and C₁₋₈ alkyl;
R^{b} is C₁₋₈ alkyl;
provided that:
   if Z is -CH₂- in the para position with respect to -OR⁴, subscript m and subscript n are 0, and R⁴ is H, then at least one R³ is selected from the group consisting of C₁₋₈ haloalkyl, halogen, -NO₂, C₂₋₈ alkyl, C₁₋₈ alkoxy, C₂₋₈ alkenyl, C₂₋₈ alkynyl, and -N(R^{a})₂;
   if Z is -CH₂- in the para position with respect to -OR⁴ and the compound is a 2,3,4-trihydroxy-benzamide, then at least one R² or at least one R³ is selected from the group consisting of -OH,-NO₂, -CN, C₁₋₈ alkoxy, C₁₋₈ haloalkyl, C₂₋₈ alkenyl, C₂₋₈ alkynyl, and -N(R^{a})₂;
   if Z is -CH₂- in the para position with respect to -OR⁴ and the compound is a 5-alkyl-6-hydroxy-benzamide, then at least one R² or at least one R³ is selected from the group consisting of -OH,-NO₂, -CN, C₁₋₈ alkyl, C₁₋₈ alkoxy, C₁₋₈ haloalkyl, C₂₋₈ alkenyl, C₂₋₈ alkynyl, and -N(R^{a})₂;
   if Z is -C(O)- in the para position with respect to -OR⁴ and the compound is a 2-hydroxy-benzamide, a 2,5-dialkyl-6-hydroxy-benzamide, or a 5-alkyl-6-hydroxy-benzamide, then (i) at least one R² or at least one R³ is selected from the group consisting of - OH, C₁₋₈ alkoxy, C₁₋₈ haloalkyl, C₂₋₈ alkenyl, C₂₋₈ alkynyl, and -N(R^{a})₂, and (ii) at least one R³ is other than -CF₃ or -NO₂ when subscript n is 1 and R² is 4-chloro or 4-(n-butyl); and
   if Z is -S- in the para position with respect to -OR⁴ and the compound is a 2,5-dialkyl-6-hydroxy-benzamide or a 5-alkyl-6-hydroxy-benzamide, then at least one R² or at least one R³ is selected from the group consisting of -OH, C₂₋₈ alkyl, C₂₋₈ alkoxy, C₂₋₈ alkenyl, C₂₋₈ alkynyl, and -N(R^{a})₂.

If Z is -CH₂- in the para position with respect to -OR⁴, subscript m and subscript n are 0, and R⁴ is H in compounds of Formula I, then at least one R³ is selected from the group consisting of halogen, -NO₂, C₂₋₈ alkyl, C₁₋₈ alkoxy, C₁₋₈ haloalkyl, C₂₋₈ alkenyl, C₂₋₈ alkynyl, and -N(R^{a})₂.

If Z is -CH₂- in the para position with respect to -OR⁴ and the compound is a 2,3,4-trihydroxy-benzamide in compounds of Formula I, then at least one R² or at least one R³ is selected from the group consisting of -OH,-NO₂, -CN, C₁₋₈ alkoxy, C₁₋₈ haloalkyl, C₂₋₈ alkenyl, C₂₋₈ alkynyl, and -N(R^{a})₂. In some such embodiments, subscript n may be 1, 2, or 3.

If Z is -CH₂- in the para position with respect to -OR⁴ and the compound is a 5-alkyl-6-hydroxy-benzamide in compounds of Formula I, then at least one R² or at least one R³ is selected from the group consisting of -OH,-NO₂, -CN, C₁₋₈ alkyl, C₁₋₈ alkoxy, C₁₋₈ haloalkyl, C₂₋₈ alkenyl, C₂₋₈ alkynyl, and -N(R^{a})₂. In some such embodiments, subscript n may be 1, 2, or 3.

If Z is -C(O)- in the para position with respect to -OR⁴ and the compound is a 2-hydroxy-benzamide, a 2,5-dialkyl-6-hydroxy-benzamide, or a 5-alkyl-6-hydroxy-benzamide in compounds of Formula I, then (i) at least one R² or at least one R³ is selected from the group consisting of -OH, C₁₋₈ alkoxy, C₁₋₈ haloalkyl, C₂₋₈ alkenyl, C₂₋₈ alkynyl, and -N(R^{a})₂, and (ii) at least one R³ is other than -CF₃ or -NO₂ when subscript n is 1 and R² is 4-chloro or 4-(n-butyl). In some such embodiments, subscript n may be 1, 2, or 3.

If Z is -S- in the para position with respect to -OR⁴ and the compound is a 2,5-dialkyl-6-hydroxy-benzamide or a 5-alkyl-6-hydroxy-benzamide in compounds of Formula I, then at least one R² or at least one R³ is selected from the group consisting of -OH, C₂₋₈ alkyl, C₂₋₈ alkoxy, C₂₋₈ alkenyl, C₂₋₈ alkynyl, and -N(R^{a})₂. In some such embodiments, subscript n may be 1, 2, or 3.

As used herein, the term "2,3,4-trihydroxy-benzamide" refers to a compound as shown below: 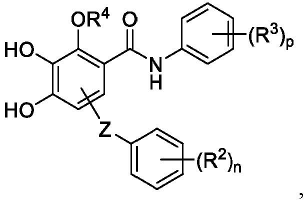 wherein R⁴ is H.

As used herein, the term "5-alkyl-6-hydroxy-benzamide" refers to a compound as shown below: 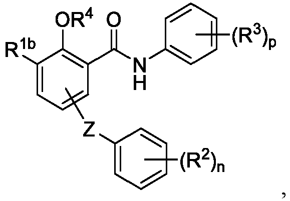 wherein R⁴ is H and R^{1b} is C₁₋₈ alkyl.

As used herein, the term "2-hydroxy-benzamide" refers to a compound as shown below: 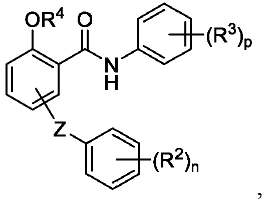 wherein R⁴ is H.

As used herein, the term "2,5-dialkyl-6-hydroxy-benzamide" refers to a compound as shown below: 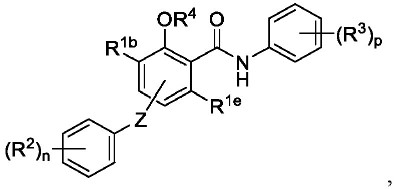 wherein R⁴ is H and R^{1b} and R^{1e} are C₁₋₈ alkyl.

In some embodiments, compounds of Formula I are provided wherein: if Z is -C(O)- in the para position with respect to -OR⁴ and the compound is a 2-hydroxy-benzamide or a 5-alkyl-6-hydroxy-benzamide, subscript n is 1, and R² is 4-chloro or 4-(*n-*butyl), then at least one R³ is other than -CF₃ or -NO₂.

In some embodiments, compounds of Formula I are provided wherein: if Z is -C(O)- in the para position with respect to -OR⁴ and the compound is a 2-hydroxy-benzamide or a 5-alkyl-6-hydroxy-benzamide, then the compound is other than 5-(4-butylbenzoyl)-2-hydroxy-*N*-[3-(trifluoromethyl)phenyl]-benzamide or 5-(4-chlorobenzoyl)-2-hydroxy-3-methyl-*N*-[4-nitro-2-(trifluoromethyl)phenyl]-benzamide.

In some embodiments, Z is -CH₂-. In some embodiments, Z is -C(O)-. In some embodiments, Z is -O-.

In some embodiments, compounds of Formula I are provided wherein each R³ is independently selected from the group consisting of -OH, halogen, -NO₂, -CN, C₁₋₈ alkyl, C₁₋₈ alkoxy, C₁₋₈ haloalkyl, C₂₋₈ alkenyl, and C₂₋₈ alkynyl.

In some embodiments, compounds of Formula I are provided wherein each R³ is independently selected from the group consisting of C₁₋₈ haloalkyl, halogen, and -NO₂.

Also provided herein are compounds according to Formula IIa: 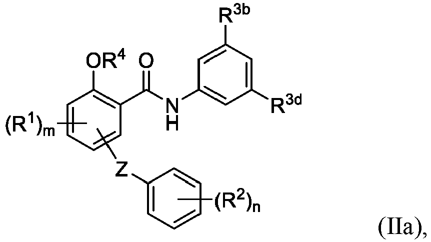 or a pharmaceutically acceptable salt thereof. In some embodiments, R^{3b} and R^{3d} are independently C₁₋₈ haloalkyl in compounds of Formula IIa. R^{3b} and R^{3d} may independently be, e.g., chloromethyl, dichloromethyl, trichloromethyl, fluoromethyl, difluoromethyl, trifluoromethyl, 2,2,2-trichloroethyl, 2,2,2-trifluoroethyl, pentachloroethyl, pentafluoroethyl, 1,1,1,3,3,3-hexachloropropyl, 1,1, 1,3,3,3-hexafluoropropyl, or the like. In some embodiments, R^{3b} and R^{3d} are independently selected from the group consisting of -CF₃ and -CCl₃.

Also provided herein are compounds according Formula IIb: 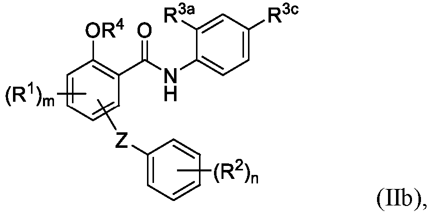 or a pharmaceutically acceptable salt thereof. In some embodiments, R^{3a} and R^{3c} are independently selected from the group consisting of halogen and -NO₂ in compounds of Formula IIb. R^{3a} and R^{3c} may independently be fluoro, chloro, bromo, iodo, or -NO₂. In some embodiments, R^{3a} is -Cl and R^{3c} is -NO₂ in compounds of Formula IIb.

Also provided herein are compounds according to Formula IIc: 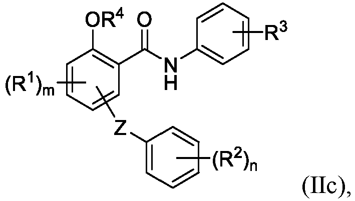 or a pharmaceutically acceptable salt thereof. In some embodiments, R³ is C₁₋₈ haloalkyl. R³ may be, e.g., chloromethyl, dichloromethyl, trichloromethyl, fluoromethyl, difluoromethyl, trifluoromethyl, 2,2,2-trichloroethyl, 2,2,2-trifluoroethyl, pentachloroethyl, pentafluoroethyl, 1,1,1,3,3,3-hexachloropropyl, 1,1, 1,3,3,3-hexafluoropropyl, or the like. In some embodiments, R³ is selected from the group consisting of -CF₃ and -CCl₃.

In some embodiments, R² is selected from the group consisting of C₁₋₈ alkyl, C₁₋₈ alkoxy, and C₁₋₈ haloalkyl in compounds of Formula I, IIa, IIb, or IIc. In some embodiments, R² is selected from the group consisting of C₁₋₈ alkyl and C₁₋₈ alkoxy, and R³ is C₁₋₈ haloalkyl.

Provided herein are compounds according to Formula IIIa: 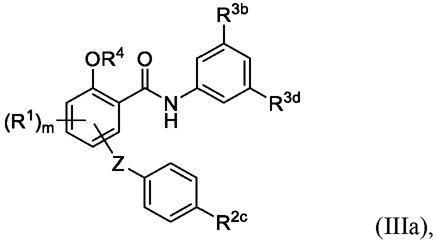 or a pharmaceutically acceptable salt thereof.

In some embodiments, R^{3b} and R^{3d} are independently C₁₋₈ haloalkyl. R^{3b} and R^{3d} may independently be, *e.g*., chloromethyl, dichloromethyl, trichloromethyl, fluoromethyl, difluoromethyl, trifluoromethyl, 2,2,2-trichloroethyl, 2,2,2-trifluoroethyl, pentachloroethyl, pentafluoroethyl, 1,1,1,3,3,3-hexachloropropyl, 1,1,1,3,3,3-hexafluoropropyl, or the like. In some embodiments, R^{3b} and R^{3d} are independently selected from the group consisting of -CF₃ and -CCl₃. In some embodiments, R² is selected from the group consisting of C₁₋₈ alkyl, C₁₋₈ alkoxy, and C₁₋₈ haloalkyl.

Provided herein are compounds according to Formula IIIb: 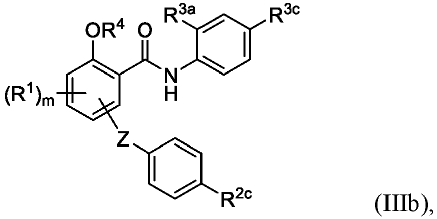 or a pharmaceutically acceptable salt thereof.

In some embodiments, R^{3a} and R^{3c} are independently selected from the group consisting of halogen and -NO₂. R^{3a} and R^{3c} may independently be fluoro, chloro, bromo, iodo, or -NO₂. In some embodiments, R^{3a} is -Cl and R^{3c} is -NO₂. In some such embodiments, R² is selected from the group consisting of C₁₋₈ alkyl, C₁₋₈ alkoxy, and C₁₋₈ haloalkyl.

Provided herein are compounds according to Formula IIIc: 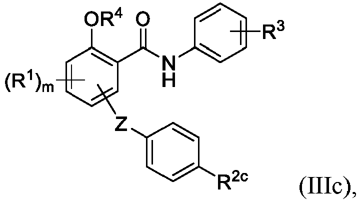 or a pharmaceutically acceptable salt thereof.

In some embodiments, R^{2c} is -OCH₃ in compounds of Formula IIIa, IIIb, or IIIc.

In some embodiments Z is present in the para position with respect to -OR⁴ in compounds of Formula I, IIa, IIb, IIc, IIIa, IIIb, or IIIc.

In some embodiments Z is present in the ortho position with respect to -C(O)NH-in compounds of Formula I, IIa, IIb, IIc, IIIa, IIIb, or IIIc.

Also provided herein are compounds according to Formula IV, V, or VI: 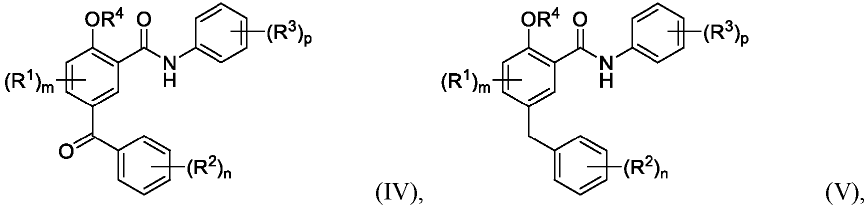 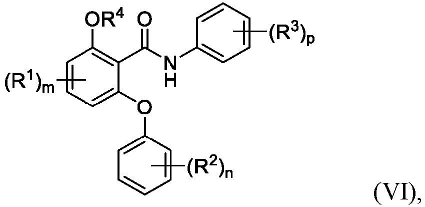 wherein R¹, R², R³, R⁴, subscript m, subscript n, and subscript p are defined as described above. Compounds according to Formula IV, V, and VI may further contain one or more of the R^{2c}, R^{3a}, R^{3b}, and R^{3c} groups set forth above.

In some embodiments, R⁴ is H or α-aminoacyl in compounds of Formula I, IIa, IIb, IIc, IIIa, IIIb, IIIc, IV, V, and/or VI. In some embodiments R⁴ is selected from the group consisting of L-valinyl and D-valinyl in compounds of Formula I, IIa, IIb, IIc, IIIa, IIIb, IIIc, IV, V, and/or VI.

In some embodiments, compounds of Formula I, IIa, IIb, IIc, IIIa, IIIb, IIIc, IV, V, and/or VI are provided wherein subscript m is 0. In some embodiments, compounds of Formula I, IIa, IIb, IIc, IV, V, and/or VI are provided wherein subscript n is 1 or 2. In some embodiments, compounds of Formula I, IV, V, and/or VI are provided wherein subscript p is 1 or 2.

In some embodiments, the compound is: 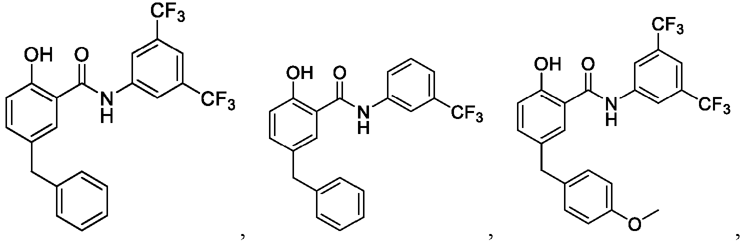 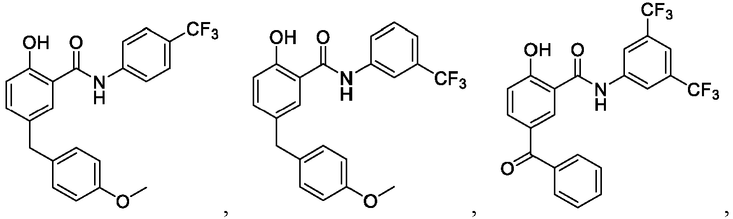 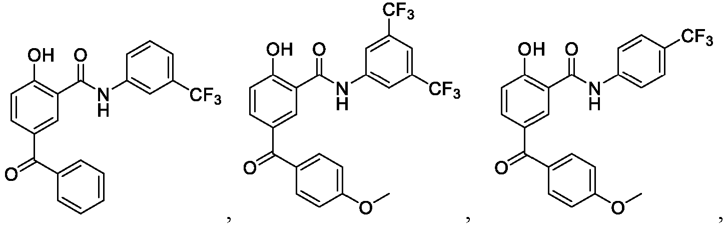 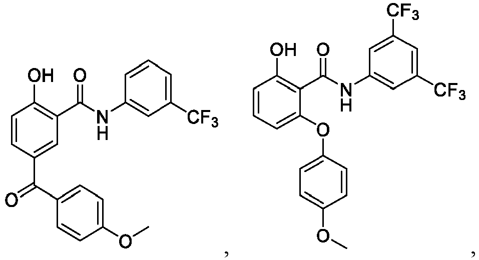 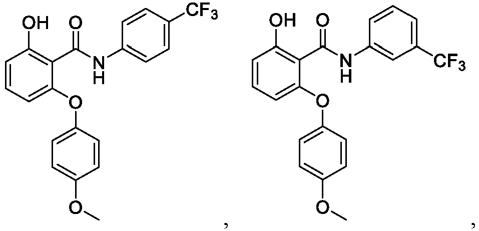 and pharmaceutically acceptable salts thereof.

In some embodiments, compounds of Formula I are provided wherein:
Z is -CH₂-;
subscript m and subscript n are 0; and
at least one R³ is selected from the group consisting of halogen, -NO₂, C₁₋₈ alkoxy, C₁₋₈ haloalkyl, C₂₋₈ alkenyl, C₂₋₈ alkynyl, and -N(R^{a})₂.

In some embodiments, compounds of Formula I are provided wherein:
Z is -C(O)- in the para position with respect to -OR⁴;
the compound is a 2-hydroxy-benzamide, a 2,5-dialkyl-6-hydroxy-benzamide, or a 5-alkyl-6-hydroxy-benzamide; and
at least one R² or at least one R³ is selected from the group consisting of -OH, C₁₋₈ alkoxy, C₂₋₈ alkenyl, C₂₋₈ alkynyl, and -N(R^{a})₂. In some such embodiments, subscript n may be 1, 2, or 3.

In some embodiments, compounds of Formula I are provided wherein:
Z is -S- in the para position with respect to -OR⁴;
the compound is a 2,5-dialkyl-6-hydroxy-benzamide or a 5-alkyl-6-hydroxy-benzamide; and
at least one R² or at least one R³ is selected from the group consisting of -OH, C₂₋₈ alkenyl, C₂₋₈ alkynyl, and -N(R^{a})₂. In some such embodiments, subscript n may be 1, 2, or 3.

In some embodiments, Z is -CH₂-, subscript m is 0, subscript n is 0, subscript p is 1, and R³ is other than 2-hydroxy, 4-cyano, 3-methyl, and 4-methyl. In some embodiments, Z is -CH₂-, subscript m is 1, R¹ is 2-methyl, subscript n is 0, subscript p is 2, a first R³ is 2-chloro, and a second R³ is other than 4-chloro. In some embodiments, Z is -CH₂-, subscript m is 2, a first R¹ is 2-hydroxy, a second R¹ is 3-hydroxy, subscript n is 1, R² is 2-isopropyl, subscript p is 1, and R³ is other than 2-chloro.

In some embodiments, Z is -S-, and the compound is other than a 2,5-dialkyl-6-hydroxy-2-methyl-benzamide or a 5-alkyl-6-hydroxy-benzamide. In some embodiments, Z is -C(O)-, and the compound is other than a 2-hydroxy-benzamide, a 2,5-dialkyl-5-hydroxy-benzamide, or a 5-alkyl-6-hydroxy-benzamide. In some embodiments, each R³ is independently selected from the group consisting of -OH, halogen, -NO₂, -CN, C₁₋₈ alkyl, C₁₋₈ alkoxy, C₁₋₈ haloalkyl, C₂₋₈ alkenyl, and C₂₋₈ alkynyl.

The compounds may be prepared using the methods disclosed herein and routine modifications thereof, which will be apparent given the disclosure herein and methods well known in the art. Synthetic routes may employ starting materials that are commercially available or those that can be prepared according to known methods, including those described in Fiesers' Reagents for Organic Synthesis Volumes 1-28 (John Wiley & Sons, 2016), by March (Advanced Organic Chemistry 6th Ed. John Wiley & Sons, 2007), and by Larock (Comprehensive Organic Transformations 3rd Ed. John Wiley & Sons, 2018). The synthesis of typical compounds described herein may be accomplished as described in the following examples. It will be appreciated that where typical or preferred process conditions (*i.e*., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures. Additionally, as will be apparent to those skilled in the art, conventional protecting groups may be advantageous for preventing certain functional groups from undergoing undesired reactions. Suitable protecting groups for various functional groups as well as suitable conditions for protecting and deprotecting particular functional groups are well known in the art. For example, numerous protecting groups are described in Green and Wuts (Protective Groups in Organic Synthesis, 4th Ed. 2007, Wiley-Interscience, New York) and references cited therein.

### III. Pharmaceutical Compositions

Also provided herein are pharmaceutical compositions comprising one or more AR/AKR1C3 inhibitors (*e*.*g*., one or more compounds according to Formula I, IIa, IIb, IIc, IIIa, IIIb, or IIIc as described above, or pharmaceutically acceptable salts thereof) and a pharmaceutically acceptable excipient.

The pharmaceutical compositions can be prepared by any of the methods well known in the art of pharmacy and drug delivery (*see, e.g.,* Lieberman, Pharmaceutical Dosage Forms (vols. 1-3, 1992); Lloyd, The Art, Science and Technology of Pharmaceutical Compounding (1999); and Remington: The Science and Practice of Pharmacy, 21st Edition, 2005, Hendrickson, Ed., Lippincott, Williams & Wilkins). In general, methods of preparing the compositions include the step of bringing one or more AR/AKR1C3 inhibitors into association with a carrier containing one or more accessory ingredients. The pharmaceutical compositions may be prepared, for example, by uniformly and intimately bringing the active ingredient into association with a liquid carrier or a finely divided solid carrier or both, and then, if necessary, shaping the product into the desired formulation. The compositions can be conveniently prepared and/or packaged in unit dosage form. Pharmaceutically acceptable carriers include any of the standard pharmaceutical carriers, buffers and excipients, including phosphate-buffered saline solution, water, and emulsions (such as an oil/water or water/oil emulsion), and various types of wetting agents and/or adjuvants. Preferred pharmaceutical carriers will depend, in part, upon the intended mode of administration of the active agent.

The pharmaceutical compositions can include a combination of drugs, *e.g.,* compounds according to Formula I, IIa, IIb, IIc, IIIa, IIIb, or IIIc, an combinations thereof, in combination with additional agents such as antiandrogen drugs (including but not limited to enzalutamide, abiraterone, bicalutamide, darolutamide, apalutamide, and the like).

The pharmaceutical compositions include those suitable for topical, parenteral, pulmonary, nasal, rectal, or oral administration. The most suitable route of administration in any given case will depend in part on the nature and severity of the condition being treated (e.g., prostate, breast, ovarian, or liver cancer, and particular stages thereof).

Other pharmaceutical compositions include those suitable for systemic (enteral or parenteral) administration. Systemic administration includes oral, rectal, sublingual, or sublabial administration. Parenteral administration includes, *e*.*g*., intravenous, intramuscular, intra-arteriole, intradermal, subcutaneous, intraperitoneal, intraventricular, and intracranial. Other modes of delivery include, but are not limited to, the use of liposomal formulations, intravenous infusion, transdermal patches, *etc.* In particular embodiments, pharmaceutical compositions may be administered intratumorally.

Compositions for pulmonary administration include, but are not limited to, dry powder compositions consisting of the powder of a compound described herein, or a salt thereof, and the powder of a suitable carrier and/or lubricant. The compositions for pulmonary administration can be inhaled from any suitable dry powder inhaler device known to a person skilled in the art.

The pharmaceutical compositions may be in a form suitable for oral use. Suitable compositions for oral administration include, but are not limited to, tablets, troches, lozenges, aqueous or oily suspensions, dispersible powders or granules, emulsions, hard or soft capsules, syrups, elixirs, solutions, buccal patches, oral gels, chewing gums, chewable tablets, effervescent powders, and effervescent tablets. Such compositions can contain one or more agents selected from sweetening agents, flavoring agents, coloring agents, antioxidants, and preserving agents in order to provide pharmaceutically elegant and palatable preparations.

Tablets generally contain the active ingredients in admixture with non-toxic pharmaceutically acceptable excipients, including: inert diluents, such as cellulose, silicon dioxide, aluminum oxide, calcium carbonate, sodium carbonate, glucose, mannitol, sorbitol, lactose, calcium phosphate, and sodium phosphate; granulating and disintegrating agents, such as corn starch and alginic acid; binding agents, such as polyvinylpyrrolidone (PVP), cellulose, polyethylene glycol (PEG), starch, gelatin, and acacia; and lubricating agents such as magnesium stearate, stearic acid, and talc. The tablets can be uncoated or coated, enterically or otherwise, by known techniques to delay disintegration and absorption in the gastrointestinal tract and thereby provide a sustained action over a longer period. For example, a time delay material such as glyceryl monostearate or glyceryl distearate can be employed. Tablets can also be coated with a semi-permeable membrane and optional polymeric osmogents according to known techniques to form osmotic pump compositions for controlled release. Compositions for oral administration can be formulated as hard gelatin capsules wherein the active ingredient is mixed with an inert solid diluent (such as calcium carbonate, calcium phosphate, or kaolin), or as soft gelatin capsules wherein the active ingredients are mixed with water or an oil medium (such as peanut oil, liquid paraffin, or olive oil).

The pharmaceutical compositions can also be in the form of an injectable aqueous or oleaginous solution or suspension. Sterile injectable preparations can be formulated using non-toxic parenterally-acceptable vehicles including water, Ringer's solution, and isotonic sodium chloride solution, and acceptable solvents such as 1,3-butane diol. In addition, sterile, fixed oils are conventionally employed as a solvent or suspending medium. For this purpose any bland fixed oil can be employed including synthetic mono- or diglycerides. In addition, fatty acids such as oleic acid find use in the preparation of injectables.

Aqueous suspensions contain the active agents in admixture with excipients suitable for the manufacture of aqueous suspensions. Such excipients include, but are not limited to: suspending agents such as sodium carboxymethylcellulose, methylcellulose, oleagino-propylmethylcellulose, sodium alginate, polyvinyl-pyrrolidone, gum tragacanth and gum acacia; dispersing or wetting agents such as lecithin, polyoxyethylene stearate, and polyethylene sorbitan monooleate; and preservatives such as ethyl, *n*-propyl, and*p-*hydroxybenzoate. Oily suspensions can be formulated by suspending the active ingredients in a vegetable oil, for example, arachis oil, olive oil, sesame oil or coconut oil, or in a mineral oil such as liquid paraffin. The oily suspensions can contain a thickening agent, for example beeswax, hard paraffin, or cetyl alcohol. These compositions can be preserved by the addition of an anti-oxidant such as ascorbic acid. Dispersible powders and granules (suitable for preparation of an aqueous suspension by the addition of water) can contain the active ingredients in admixture with a dispersing agent, wetting agent, suspending agent, or combinations thereof. Additional excipients can also be present.

The pharmaceutical compositions can also be in the form of oil-in-water emulsions. The oily phase can be a vegetable oil, for example olive oil or arachis oil, or a mineral oil, for example liquid paraffin or mixtures of these. Suitable emulsifying agents can be naturally-occurring gums, such as gum acacia or gum tragacanth; naturally-occurring phospholipids, such as soy lecithin; esters or partial esters derived from fatty acids and hexitol anhydrides, such as sorbitan monooleate; and condensation products of said partial esters with ethylene oxide, such as polyoxyethylene sorbitan monooleate.

Transdermal delivery can be accomplished by means of iontophoretic patches and the like. The active ingredients can also be administered in the form of suppositories for rectal administration of the drug. These compositions can be prepared by mixing the active agents with a suitable non-irritating excipient which is solid at ordinary temperatures but liquid at the rectal temperature and will therefore melt in the rectum to release the drug. Such materials include cocoa butter and polyethylene glycols.

Controlled release parenteral formulations of the compositions can be made as implants, oily injections, or as particulate systems. For a broad overview of delivery systems see Banga, A.J., THERAPEUTIC PEPTIDES AND PROTEINS: FORMULATION, PROCESSING, AND DELIVERY SYSTEMS, Technomic Publishing Company, Inc., Lancaster, PA, (1995). Particulate systems include microspheres, microparticles, microcapsules, nanocapsules, nanospheres, and nanoparticles.

Polymers can be used for ion-controlled release of active agents. Various degradable and nondegradable polymeric matrices for use in controlled drug delivery are known in the art (Langer R., Accounts Chem. Res., 26:537-542 (1993)). For example, the block copolymer, polaxamer 407 exists as a viscous yet mobile liquid at low temperatures but forms a semisolid gel at body temperature. It has shown to be an effective vehicle for formulation and sustained delivery of recombinant interleukin 2 and urease (Johnston et al., Pharm. Res., 9:425-434 (1992); and Pec et al., J. Parent. Sci. Tech., 44(2):58 65 (1990)). Alternatively, hydroxyapatite has been used as a microcarrier for controlled release of proteins (Ijntema et al., Int. J. Pharm., 112:215-224 (1994)). In yet another aspect, liposomes are used for controlled release as well as drug targeting of the lipid-capsulated drug (Betageri et al., LIPOSOME DRUG DELIVERY SYSTEMS, Technomic Publishing Co., Inc., Lancaster, PA (1993)). Numerous additional systems for controlled delivery of therapeutic proteins are known. *See, e.g.,* U.S. Pat. No. 5,055,303, 5,188,837, 4,235,871, 4,501,728, 4,837,028 4,957,735 and 5,019,369, 5,055,303; 5,514,670; 5,413,797; 5,268,164; 5,004,697; 4,902,505; 5,506,206, 5,271,961; 5,254,342 and 5,534,496.

### IV. Methods of Treating Androgen-Mediated Diseases

Any references to methods of treatment by therapy or surgery or in vivo diagnosis methods in this description are to be interpreted as references to compounds, pharmaceutical compositions and medicaments described herein for use in those methods.
Also provided herein is a compound according to any one of Formulas I, IIa, IIb, IIc, IIIa, IIIb, or IIIc or a pharmaceutically acceptable salt thereof, or a pharmaceutically acceptable composition as described above for use in methods for treating an androgen-mediated disease or condition, the method comprising administering to a subject in need thereof a therapeutically effective amount of the compound according to any one of Formulas I, IIa, IIb, IIc, IIIa, IIIb, or IIIc or a pharmaceutically acceptable salt thereof, or a therapeutically effective amount of the pharmaceutically acceptable composition as described above, thereby treating the hormone-mediated disease or condition.

In some embodiments, the hormone-mediated disease is a cancer. The cancer may be, for example, an androgen-independent cancer, a metastatic cancer, a castrate-resistant cancer, a castration recurrent cancer, a hormone-resistant cancer, a metastatic castrate-resistant cancer, or a combination thereof. Compounds according to the present disclosure can be used for treating carcinomas of the breast, prostate, endometrium, or kidney, as well as hepatocellular carcinoma, bladder cancers, renal cancers, gastric cancers, cervical cancers, colon cancers, and lung cancers (*e*.*g*., non-small cell lung cancer; NSCLC). These and other cancers are known to express AKR1C3. *See, e.g.,* Guise et al. Cancer Res 2010 (70) (4) 1573-1584. In some embodiments, the cancer is prostate cancer, breast cancer, ovarian cancer, or liver cancer.

In some embodiments, the method includes administering an antiandrogen agent to the subject. The antiandrogen agent is selected from the group consisting of enzalutamide, apalutamide, darolutamide, abiraterone, pharmaceutically acceptable salts thereof, and combinations thereof.

The compounds and/or pharmaceutical compositions as described herein can be administered at any suitable dose in the methods. In general, the compound and/or composition is administered at a dose ranging from about 0.1 milligrams to about 1000 milligrams per kilogram of a subject's body weight (*i.e.,* about 0.1-1000 mg/kg). In some embodiments, the compound and/or composition is administered at a dose ranging from about 1 milligram to about 100 milligrams per kilogram of a subject's body weight (*i.e.,* about 1-100 mg/kg). The dose can be, for example, about 0.1-1000 mg/kg, or about 1-10 mg/kg, or about 10-50 mg/kg, or about 25-50 mg/kg, or about 50-75 mg/kg, or about 1-75-100 mg/kg, or about 1-500 mg/kg, or about 25-250 mg/kg, or about 50-100 mg/kg. The dose can be about 1, 2, 3, 4, 5, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950 or 1000 mg/kg. The dosages can be varied depending upon the requirements of the patient, the severity of the disorder being treated, and the particular formulation being administered. The dose administered to a patient should be sufficient to result in a beneficial therapeutic response in the patient. The size of the dose will also be determined by the existence, nature, and extent of any adverse side-effects that accompany the administration of the drug in a particular patient. Determination of the proper dosage for a particular situation is within the skill of the typical practitioner. The total dosage can be divided and administered in portions over a period of time suitable to treat to the cancer or other disease/condition.

The compounds and/or compositions can be administered for periods of time which will vary depending upon the nature of the particular disorder, its severity, and the overall condition of the subject to whom the compounds and/or compositions are administered. Administration can be conducted, for example, hourly, every 2 hours, three hours, four hours, six hours, eight hours, or twice daily including every 12 hours, or any intervening interval thereof. Administration can be conducted once daily, or once every 36 hours or 48 hours, or once every month or several months. Following treatment, a subject can be monitored for changes in his or her condition and for alleviation of the symptoms of the disorder. The dosage can either be increased in the event the subject does not respond significantly to a particular dosage level, or the dose can be decreased if an alleviation of the symptoms of the disorder is observed, or if the disorder has been remedied, or if unacceptable side effects are seen with a particular dosage. A therapeutically effective amount can be administered to the subject in a treatment regimen comprising intervals of at least 1 hour, or 6 hours, or 12 hours, or 24 hours, or 36 hours, or 48 hours between dosages. Administration can be conducted at intervals of at least 72, 96, 120, 144, 168, 192, 216, or 240 hours (*i.e.,* 3, 4, 5, 6, 7, 8, 9, or 10 days).

In some embodiments, the methods further include administration of one or more additional anti-cancer agents. Examples of anti-cancer agents include, but are not limited to, chemotherapeutic agents (*e.g*., carboplatin, paclitaxel, pemetrexed, or the like), tyrosine kinase inhibitors (*e.g*., erlotinib, crizotinib, osimertinib, or the like), poly (ADP-ribose) polymerase inhibitors (*e.g*., olaparib, rucaparib, and the like), and immunotherapeutic agents (*e.g*., pembrolizumab, nivolumab, durvalumab, atezolizumab, or the like). In some embodiments, the methods include administration of radiotherapy, *e.g*., external beam radiation; intensity modulated radiation therapy (IMRT); brachytherapy (internal or implant radiation therapy); stereotactic body radiation therapy (SBRT)/stereotactic ablative radiotherapy (SABR); stereotactic radiosurgery (SRS); or a combination of such techniques.

In some of these embodiments, the cancer is advanced stage cancer. In some of these embodiments, the cancer is drug resistant. In some of these embodiments, the cancer is antiandrogen drug resistant or androgen independent. In some of these embodiments, the cancer is metastatic. In some of these embodiments, the cancer is metastatic and drug resistant (*e.g*., antiandrogen drug resistant). In some of these embodiments, the cancer is castration resistant. In some of these embodiments, the cancer is metastatic and castration resistant. In some of these embodiments, the cancer is enzalutamide resistant. In some of these embodiments, the cancer is enzalutamide and arbiraterone resistant. In some of these embodiments, the cancer is enzalutamide, arbiraterone, darolutamide, and bicalutamide resistant. In some of these embodiments, the cancer is enzalutamide, arbiraterone, bicalutamide, darolutamide, and apalutamide resistant. In other embodiments, the cancer is resistant (*e.g.,* docetaxel, cabazitaxel, paclitaxel). The cancer (*e.g.,* prostate, breast, ovarian, or liver cancer) can be resistant to any combination of these drugs.

In some embodiments, treatment comprises inhibiting cancer cell (*e.g.,* prostate, breast, ovarian, or liver cancer cell) growth, inhibiting cancer cell proliferation, inhibiting cancer cell migration, inhibiting cancer cell invasion, ameliorating the symptoms of cancer, reducing the size of a cancer tumor, reducing the number of cancer tumors, reducing the number of cancer cells, inducing cancer cell necrosis, pyroptosis, oncosis, apoptosis, autophagy, or other cell death, or enhancing the therapeutic effects of a composition or pharmaceutical composition comprising a AR/AKR1C3 inhibitor. In particular instances, the subject does not have cancer.

In particular methods of treating cancer (*e.g*., prostate cancer, breast cancer, ovarian cancer, liver cancer, androgen-independent cancer, or drug-resistant cancer), described herein, treatment comprises enhancing the therapeutic effects of an antiandrogen drug (*e.g.,* a non-steroidal adrogen recept antagonist or a CYP17A1 inhibitor). In certain embodiments, treatment comprises enhancing the therapeutic effects of enzalutamide. In certain other embodiments, treatment comprises enhancing the therapeutic effects of abiraterone. In yet other embodiments, treatment comprises enhancing the therapeutic effects of apalutamide. In some other embodiments, treatment comprises enhancing the therapeutic effects of bicalutamide. The enhancement can be synergistic or additive.

In certain embodiments of the methods set forth herein, treatment comprises reversing, reducing, or decreasing cancer cell (*e.g*., prostate cancer cell, breast cancer cell, ovarian cancer cell, or liver cancer cell) resistance to antiandrogen drugs. In certain embodiments of the methods set forth herein, treatment comprises resensitizing cancer cells (*e*.*g*., prostate cancer cells or breast cancer cells) to antiandrogen drugs. In any of the methods described herein, the antiandrogen drug is a compound selected from the group consisting of a non-steroidal androgen receptor antagonist, a CYP17A1 inhibitor, and a combination thereof. In certain embodiments, the antiandrogen drug is enzalutamide, apalutamide, bicalutamide, and/or abiraterone acetate

In any of the aforementioned methods, treatment may comprise reversing cancer cell (*e.g.,* prostate, breast, ovarian, or liver cancer cell) resistance to an antiandrogen drug (*e.g*., a non-steroidal androgen receptor antagonist or CYP17A1 inhibitor); reducing or decreasing cancer cell resistance to an antiandrogen drug; or resensitizing cancer cells to an antiandrogen drug. In some embodiments, treatment comprises reversing cancer cell (*e.g*., prostate, breast, ovarian, or liver cancer cell) resistance to enzalutamide, apalutamide, bicalutamide, darolutamide, abiraterone acetate, or a combination thereof. In some other embodiments, treatment comprises reducing or decreasing cancer cell resistance to enzalutamide, apalutamide, bicalutamide, darolutamide, abiraterone acetate, or a combination thereof. In some embodiments, treatment comprises resensitizing cancer cells to enzalutamide, apalutamide, bicalutamide, abiraterone acetate, darolutamide, or a combination thereof.

In any of the methods described herein, the cancer is selected from the group consisting of castration-resistant cancer, metastatic castration-resistant cancer, advanced stage cancer, drug-resistant cancer, antiandrogen-resistant cancer, bicalutamide resistant cancer, enzalutamide-resistant cancer, abiraterone acetate-resistant cancer, apalutamide-resistant cancer, darolutamide-resistant cancer, AR-V1-, AR-V3-, AR-V7-, AR-V9-, and/or AR-V12-induced drug-resistant cancer, AR-V1-, AR-V3-, AR-V7-, AR-V9-, and/or AR-V12-induced antiandrogen drug-resistant cancer, AR-V1-, AR-V3-, AR-V7-, AR-V9-, and/or AR-V12-induced enzalutamide-resistant cancer, AR-V1-, AR-V3-, AR-V7-, AR-V9-, and/or AR-V12-induced abiraterone acetate-resistant cancer, AR-V1-, AR-V3-, AR-V7-, AR-V9-, and/or AR-V12-induced apalutamide-resistant cancer, AR-V1-, AR-V3-, AR-V7-, AR-V9-, and/or AR-V12-induced bicalutamide-resistant cancer, and combinations thereof.

In some embodiments, a test sample is obtained from the subject. The test sample can be obtained before and/or after the AR/AKR1C3 inhibitor(s) is administered to the subject. Non-limiting examples of suitable samples include blood, serum, plasma, cerebrospinal fluid, tissue, saliva, and urine. In some instances, the sample comprises normal tissue. In other instances, the sample comprises cancer tissue. The sample can also be made up of a combination of normal and cancer cells.

In some embodiments, a reference sample is obtained. The reference sample can be obtained, for example, from the subject and can comprise normal tissue. The reference sample can be also be obtained from a different subject and/or a population of subjects. In some instances, the reference sample is either obtained from the subject, a different subject, or a population of subjects before and/or after the AR/AKR1C3 inhibitor(s) is administered to the subject, and comprises normal tissue. However, in some instances the reference sample comprises cancer tissue and is obtained from the subject and/or from a different subject or a population of subjects.

In some embodiments, the level of one or more biomarkers is determined in the test sample and/or reference sample. Non-limiting examples of suitable biomarkers include prostate-specific antigen (PSA), alpha-methylacyl-CoA racemase (AMACR), endoglin (CD105), engrailed 2 (EN-2), prostate-specific membrane antigen (PSMA), caveolin-1, interleukin-6 (IL-6), CD147, members of the S100 protein family (*e.g*., S100A2, S100A4, S100A8, S100A9, S100A11), annexin A3 (ANXA3), human kallikrein-2 (KLK2), TGF-Beta1, beta-microseminoprotein (MSMB), estrogen receptor (ER), progesterone receptor (PgR), HER2, Ki67, cyclin D1, and cyclin E.

Prostate-specific antigen (PSA) is a protein produced primarily by prostate cells. Most PSA is released into the semen, but some PSA is also released into the blood. In the blood, PSA exists in unbound and complexed (cPSA) forms. Conventional laboratory tests can measure unbound and/or total (unbound and complexed) PSA. Elevated PSA levels can be caused by benign prostatic hyperplasia (BPH) and inflammation of the prostate, but can also be caused by prostate cancer. Determining PSA levels may also include one or more determinations of PSA velocity (*i.e.,* the change in PSA level over time), PSA doubling time (*i.e.,* how quickly the PSA level doubles), PSA density (*i.e.,* a comparison of the PSA concentration and the volume of the prostate (which can be evaluated, for example, by ultrasound)), and age-specific PSA ranges.

Typically, the level of the one or more biomarkers in one or more test samples is compared to the level of the one or more biomarkers in one or more reference samples. Depending on the biomarker, and increase or a decrease relative to a normal control or reference sample can be indicative of the presence of cancer or a higher risk for cancer. As a non-limiting example, levels of one or biomarkers in test samples taken before and after the AR/AKR1C3 inhibitor(s) is administered to the subject are compared to the level of the one or more biomarkers in a reference sample that is either normal tissue obtained from the subject, or normal tissue that is obtained from a different subject or a population of subjects. In some instances, the biomarker is serum, and the level of PSA in a test sample obtained from the subject before the AR/AKR1C3 inhibitor(s) is administered to the subject is higher than the level of PSA in the reference sample. In other instances, the level of PSA in a test sample obtained from the subject after administration of the AR/AKR1C3 inhibitor(s) is decreased relative to the level of PSA in a test sample obtained prior to administration. Alternatively, as another non-limiting example, the difference in PSA level between a sample obtained from the subject after administration and a reference sample is smaller than a difference between the PSA level in a sample obtained from the subject prior to administration and the reference sample (*i.e*., administration results in a decrease in PSA in the test sample such that the difference between the level measured in the test sample and the level measured in the reference sample is diminished or eliminated).

The differences between the reference sample or value and the test sample need only be sufficient to be detected. In some embodiments, an increased level of a biomarker (*e.g*., PSA) in the test sample, and hence the presence of cancer or increased risk of cancer, is determined when the biomarker levels are at least, e.g., about 1-fold, 2-fold, 3-fold, 4-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, or 20-fold higher in comparison to a negative control. In other embodiments, a decreased level of a biomarker in the test sample, and hence the presence of cancer or increased risk of cancer, is determined when the biomarker levels are at least, *e.g.,* about 1-fold, 2-fold, 3-fold, 4-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, or 20-fold lower in comparison to a negative control.

The biomarker levels can be detected using any method known in the art, including the use of antibodies specific for the biomarkers. Exemplary methods include, without limitation, PCR, Western Blot, dot blot, enzyme-linked immunosorbent assay (ELISA), radioimmunoassay (RIA), immunoprecipitation, immunofluorescence, FACS analysis, electrochemiluminescence, and multiplex bead assays (*e*.*g*., using Luminex or fluorescent microbeads). In some instances, nucleic acid sequencing is employed.

In certain embodiments, the presence of decreased or increased levels of one or more biomarkers is indicated by a detectable signal (*e.g.,* a blot, fluorescence, chemiluminescence, color, radioactivity) in an immunoassay or PCR reaction (*e*.*g*., quantitative PCR). This detectable signal can be compared to the signal from a control sample or to a threshold value. In some embodiments, a decreased presence is detected, and the presence or increased risk of cancer is indicated, when the detectable signal of biomarker(s) in the test sample is at least, *e*.*g*., about 1-fold, 2-fold, 3-fold, 4-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, or 20-fold lower in comparison to the signal of antibodies in the reference sample or the predetermined threshold value. In other embodiments, an increased presence is detected, and the presence or increased risk of cancer is indicated, when the detectable signal of biomarker(s) in the test sample is at least, *e*.*g*., about 1-fold, 2-fold, 3-fold, 4-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, or 20-fold greater in comparison to the signal of antibodies in the reference sample or the predetermined threshold value.

Also described herein is a method for inhibiting an androgen receptor (AR), the method comprising contacting the AR with an effective amount of a compound as described herein (*e.g.,* a compound according to Formula I, IIa, IIb, IIc, IIIa, IIIb, or IIIc) or a salt thereof, thereby inhibiting the AR.

Also described herein is a method for inhibiting aldo-keto reductase family 1 member C3 (AKR1C3), the method comprising contacting the AKR1C3 with an effective amount of a compound as described herein (*e.g.,* a compound according Formula I, IIa, IIb, IIc, IIIa, IIIb, or IIIc) or a salt thereof, thereby inhibiting the AKR1C3.
Inhibiting the AR or the AKR1C3 generally includes contacting the AR or the AKR1C3 with an amount of the compound sufficient to reduce the activity of the AR or the AKR1C3 as compared to activity of the AR or the AKR1C3 in the absence of the compound. For example, contacting the AR or the AKR1C3 with the inhibitor can result in from about 1% to about 99% inhibition (*i.e.,* the activity of the inhibited AR or AKR1C3 ranges from 99% to 1% of the AR activity or AKR1C3 activity in the absence of the compound). The level of inhibition can range from about 1% to about 10%, or from about 10% to about 20%, or from about 20% to about 30%, or from about 30% to about 40%, or from about 40% to about 50%, or from about 50% to about 60%, or from about 60% to about 70%, or from about 70% to about 80%, or from about 80% to about 90%, or from about 90% to about 99%. The level of inhibition can range from about 5% to about 95%, or from about 10% to about 90%, or from about 20% to about 80%, or from about 30% to about 70%, or from about 40% to about 60%. In some embodiments, contacting the AR or the AKR1C3 with a compound as described herein will result in complete (*i.e.,* 100%) inhibition of the AR or the AKR1C3.

### V. Kits

Compounds as described herein are useful in kits for preventing or treating cancer in a subject. The kits are useful for treating any cancer, some non-limiting examples of which include prostate cancer, breast cancer, uterine cancer, ovarian cancer, liver cancer, colorectal cancer, stomach cancer, pancreatic cancer, lung cancer (*e*.*g*., mesothelioma, lung adenocarcinoma), esophageal cancer, head and neck cancer, sarcomas, melanomas, thyroid carcinoma, CNS cancers (*e*.*g*., neuroblastoma, glioblastoma), chronic lymphocytic leukemia, and any other cancer described herein. The kits are also suitable for treating androgen-independent, castrate-resistant, castration recurrent, hormone-resistant, drug-resistant, and metastatic castrate-resistant cancers.

In some embodiments, the kits comprise an AR/AKR1C3 inhibitor. In some other embodiments, the kits further comprise a pharmaceutically acceptable carrier. In particular embodiments, the AR/AKR1C3 inhibitor is a compound according to Formulas I, IIa, IIb, IIc, IIIa, IIIb, and/or IIIc.

In some embodiments, the antiandrogen drug is a non-steroidal androgen receptor antagonist, a CYP17A1 inhibitor, or a combination thereof. Suitable non-steroidal AR antagonists include bicalutamide (Casodex, Cosudex, Calutide, Kalumid), flutamide, nilutamide, apalutamide (ARN-509, JNJ-56021927), darolutamide, enzalutamide (Xtandi), cimetidine and topilutamide. Suitable CYP17A1 inhibitors include abiraterone acetate (Zytiga), ketoconazole, and seviteronel. Any combination of antiandrogen drugs can be used in the kits.

Materials and reagents to carry out the various methods described above can be provided in kits to facilitate execution of the methods. As used herein, the term "kit" includes a combination of articles that facilitates a process, assay, analysis, or manipulation. The kits may be utilized in a wide range of applications including, for example, diagnostics, prognostics, therapy, and the like.

Kits can contain chemical reagents as well as other components. In addition, the kits can include, without limitation, instructions to the kit user, apparatus and reagents for sample collection and/or purification, apparatus and reagents for product collection and/or purification, apparatus and reagents for administering AR/AKR1C3 inhibitor(s), apparatus and reagents for determining the level(s) of biomarker(s), sample tubes, holders, trays, racks, dishes, plates, solutions, buffers or other chemical reagents, suitable samples to be used for standardization, normalization, and/or control samples. Kits can also be packaged for convenient storage and safe shipping, for example, in a box having a lid.

In some embodiments, the kits also contain negative and positive control samples for detection of biomarkers. Non-limiting examples of suitable biomarkers include prostate-specific antigen (PSA), alpha-methylacyl-CoA racemase (AMACR), endoglin (CD105), engrailed 2 (EN-2), prostate-specific membrane antigen (PSMA), caveolin-1, interleukin-6 (IL-6), CD147, members of the S100 protein family (*e*.*g*., S100A2, S100A4, S100A8, S100A9, S100A11), annexin A3 (ANXA3), human kallikrein-2 (KLK2), TGF-Beta1, beta-microseminoprotein (MSMB), estrogen receptor (ER), progesterone receptor (PgR), HER2, Ki67, cyclin D1, and cyclin E. In some instances, the one or more biomarkers comprises PSA. In some embodiments, the negative control samples are obtained from individuals or groups of individuals who do not have cancer. In other embodiments, the positive control samples are obtained from individuals or groups of individuals who have cancer. In some embodiments, the kits contain samples for the preparation of a titrated curve of one or more biomarkers in a sample, to assist in the evaluation of quantified levels of the one or more biomarkers in a test biological sample.

### VI. Examples

### Example 1. Synthesis of dual AR/AKR1C3 inhibitors.

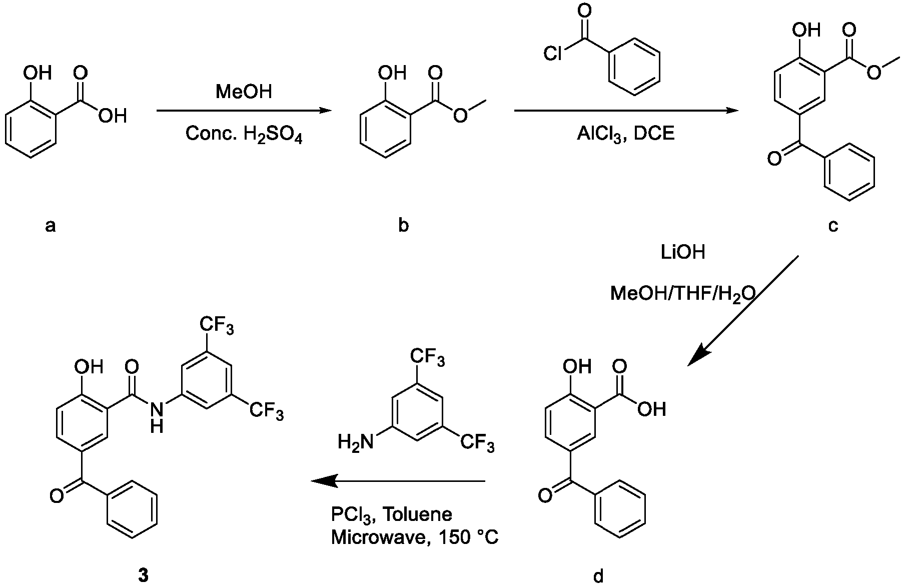

5-Benzoyl-N-(3,5-bis(trifluoromethyl)phenyl)-2-hydroxybenzamide (3) was prepared as summarized in the scheme above. 2-Hydroxybenzoic acid (a) was esterified to yield methyl ester (b). Acylation of (b) afforded benzophenone (c) which was subsequently hydrolyzed to yield benzoic acid (d). Microwave-assisted amidation of (d) afforded compound 3. ¹H NMR (400 MHz, DMSO-*d*₆) δ 12.05 (s, br, 1H), 11.00 (s, 1H), 8.46 (s, 2H), 8.27 (d, *J* = 2.3 Hz, 1H), 7.92 - 7.81 (m, 2H), 7.78 - 7.64 (m, 3H), 7.58 (t, *J =* 7.5 Hz, 2H), 7.17 (d, *J =* 8.6 Hz, 1H).

N-(3,5-Bis(trifluoromethyl)phenyl)-2-hydroxy-5-(4-methoxybenzoyl)benzamide (8) was prepared as summarized in the following scheme. 2-Hydroxybenzoic acid (a) was esterified to yield methyl ester (b). Acylation of (b) afforded benzophenone (e) which was subsequently hydrolyzed to yield benzoic acid (f). Microwave assisted-amidation of (f) afforded compound **8.** ¹H NMR (300 MHz, DMSO-*d*₆) δ 11.98 (s, br, 1H), 10.93 (s, 1H), 8.45 (d, *J=* 1.6 Hz, 2H), 8.24 (d, *J=* 2.2 Hz, 1H), 7.90 - 7.70 (m, 4H), 7.13 (dd, *J=* 17.8, 8.7 Hz, 3H), 3.87 (s, 3H).

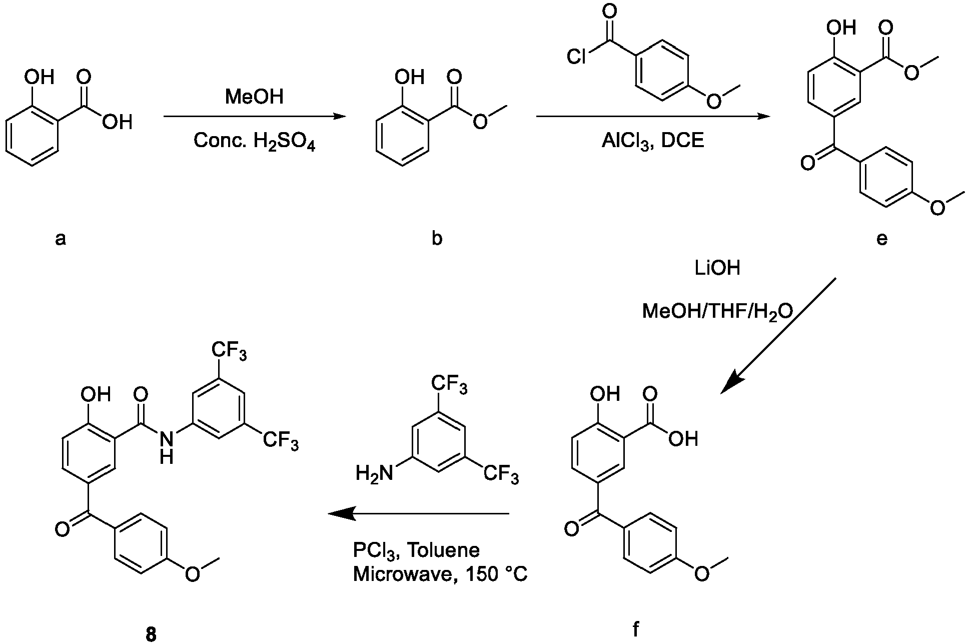

*N*-(3,5-bis(trifluoromethyl)phenyl)-2-hydroxy-5-(4-methoxybenzyl)benzamide **(5)** and 5-benzyl-*N*-(3,5-bis(trifluoromethyl)phenyl)-2-hydroxybenzamide (1) were prepared as summarized in the following scheme. Compound **8** and compound **3** were reduced to afford compound **5** and compound **1,** respectively. Compound **1:** ¹H NMR (300 MHz, DMSO-*d*₆) δ 11.16 (s, 1H), 10.81 (s, 1H), 8.51 - 8.42 (m, 2H), 7.87 - 7.80 (m, 1H), 7.74 (d, *J =* 2.3 Hz, 1H), 7.35 - 7.16 (m, 6H), 6.95 (d, *J =* 8.4 Hz, 1H), 3.92 (s, 2H).

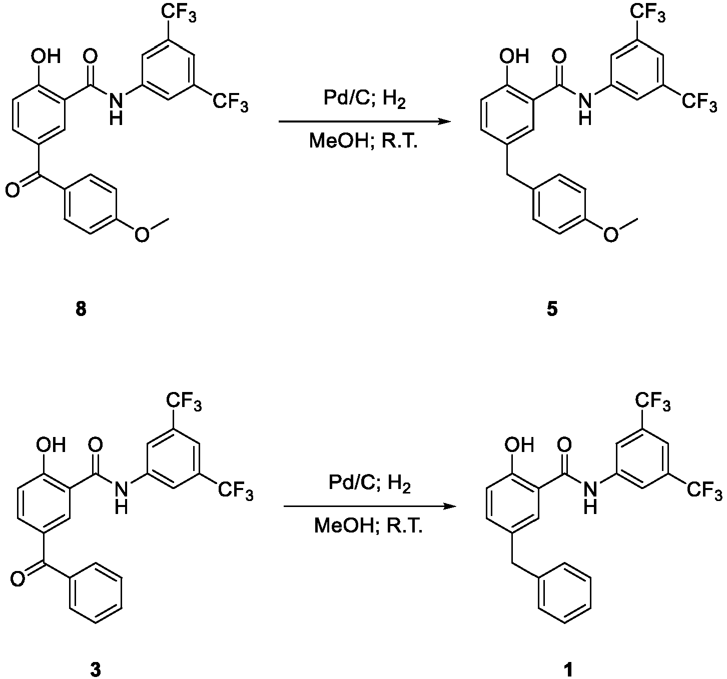

2-Hydroxy-5-(4-methoxybenzyl)-N-(4-(trifluoromethyl)phenyl)benzamide **(6)** was prepared in similar fashion. ¹H NMR (300 MHz, DMSO-*d*₆) δ 11.31 (s, 1H), 10.61 (s, 1H), 7.93 (d, *J=* 8.4 Hz, 2H), 7.78 - 7.68 (m, 3H), 7.26 (dd, *J =* 8.4, 2.3 Hz, 1H), 7.20 - 7.09 (m, 2H), 6.98 - 6.80 (m, 3H), 3.85 (s, 2H), 3.71 (s, 3H).

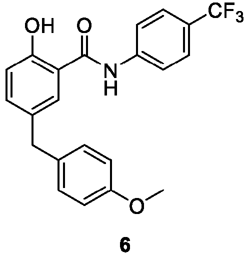

Synthesis of *N*-(3,5-bis(trifluoromethyl)phenyl)-2-hydroxy-6-(4-methoxyphenoxy)benzamide **(39)** was prepared as summarized in the following scheme. 2-Fluoro-6-hydroxybenzaldehyde (g) was converted to the corresponding ether-protected compound (h). Coupling of compound (h) with 4-methoxyphenol afforded oxydibenzene (i) which was subsequently oxidized to yield benzoic acid (j). Further reduction of (j) afforded the hydroxybenzoic acid (k). Final microwave assisted amidation of (k) yielded the 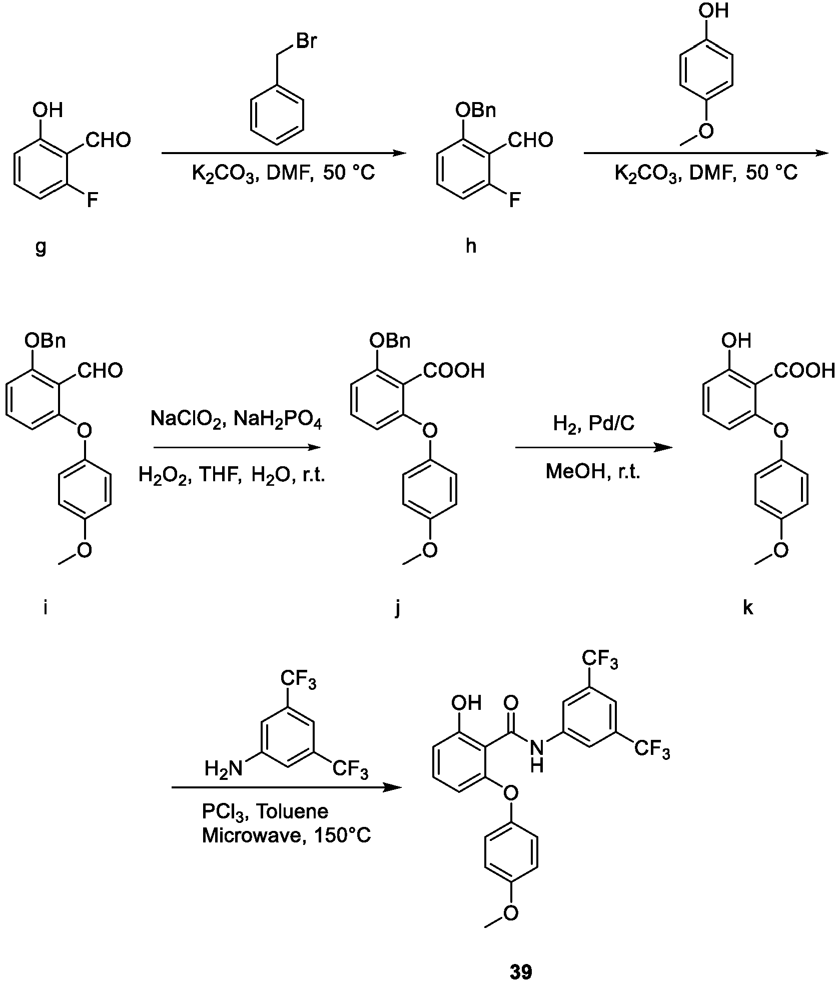 compound **39.** ¹H NMR (300 MHz, DMSO-*d*₆) δ 11.06 (s, 1H), 10.22 (s, 1H), 8.41 - 8.34 (m, 2H), 7.78 (dq, *J =* 1.8, 0.9 Hz, 1H), 7.17 (t, *J =* 8.3 Hz, 1H), 7.06 - 6.89 (m, 4H), 6.67 (dd, *J* = 8.3, 0.8 Hz, 1H), 6.19 (dd, *J =* 8.3, 0.8 Hz, 1H), 3.72 (s, 3H).

### Example 2. Inhibition of AR/AR-Vs and AKR1C3 expression.

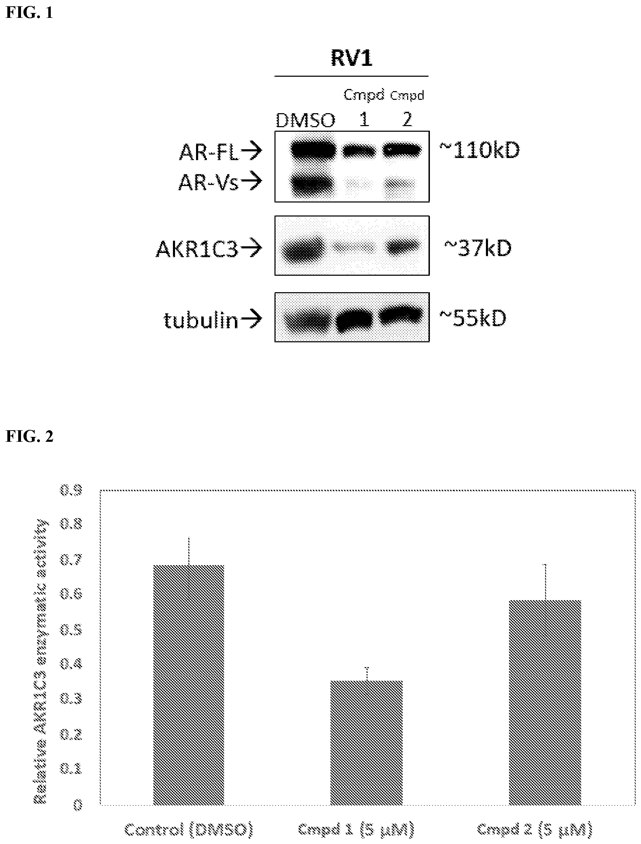

To determine the ability of compound **1** and compound **2** to inhibit the expression of AR/AR-Vs and AKR1C3 expression CWR22rv1 cells were treated with the two compounds. The cells were treated with 5 µM compound **1** or 5 µM compound **2** for 48 hrs. Following treatment, cell lysates were collected and analyzed. Analysis was performed using Western blot analysis for AR-FL, AR-Vs, and AKR1C3 protein expression. The results of this analysis is shown in the Western blot images in FIG. 1.

### Example 3. Inhibition of AKR1C3 activity.

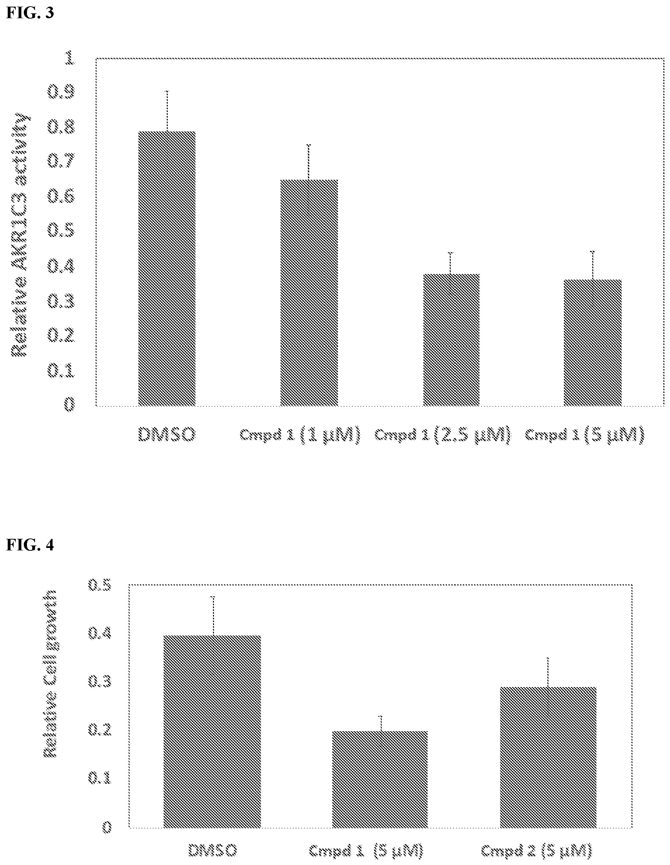

To examine whether compound **1** and compound **2** are useful in inhibiting AKR1C3 activity, LNCaP-AKR1C3 were treated with either compound **1** or compound **2** for 48 hrs. AKR1C3 enzymatic activity was determined by Aldo-Keto Reductase (AKR) Activity Assay Kit (Colorimetric), the results of which are shown in FIG. 2. Similarly, the inhibition of AKR1C3 activity was examined with varying doses of compound **1.** LNCaP-AKR1C3 cells were treated with increasing doses of compound **1** for 48 hrs. Analysis was performed using a AKR-Activity Assay Kit (colorimetric), the results of which are shown in FIG. 3.

### Example 4. Inhibition of enzalutamide-resistant prostate cancer cell growth.

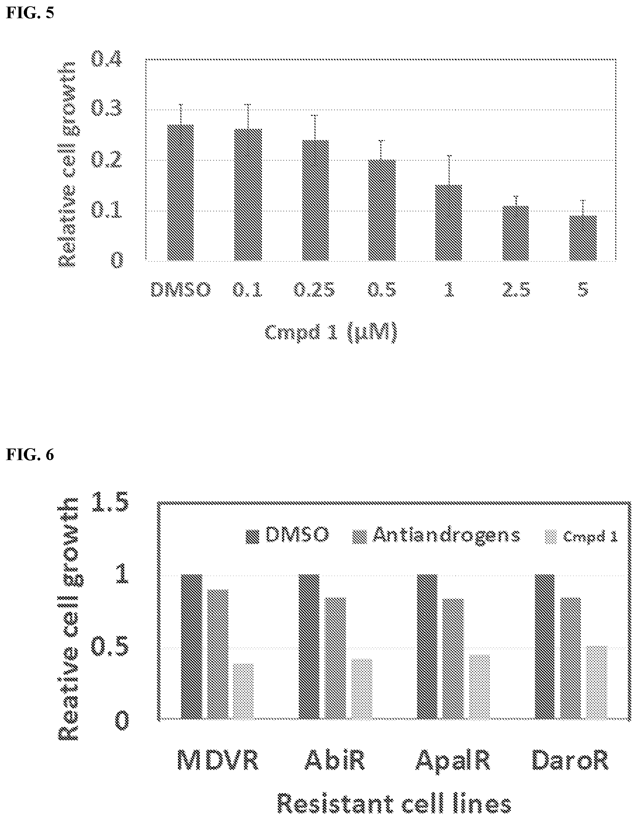

To examine to ability of the compounds to inhibit cell growth, C4-2B MDVR cells were treated with either compound **1** or compound **2** for 48 hrs. The cell number was determined and graphically summarized in FIG. 4. Similarly, the dose-dependent inhibition of cell growth was examined. C4-2B MDVR cells were treated with increasing doses of compound **1** for 48 hrs. The cell number was determined at each dose of compound **1** and graphically summarized in FIG. 5.

### Example 5. Inhibition of cancer cell growth with AR/AKR1C3 inhibitors and antiandrogens.

To evaluate whether compound **1** is an effective inhibitor of anti-androgen resistant cell growth, a series of resistant cells were treated with their respective anti-androgens for 48 hrs. Enzalutamide resistant MDVR cells, abiraterone resistant AbiR cells, apalutamide resistant ApaIR cells, and darolutamide resistant DaroR cells were treated with their respective anti-androgens: enza (20 µM), abi (20 µM), apal (20 µM), darolutamide (20 µM), or compound **1** (5 µM). The cell number was determined for each resistant cell type and graphically summarized in FIG. 6.

### Example 6. Inhibition of prostate cancer cell growth in vivo with AR/AKR1C3 inhibitor 1.

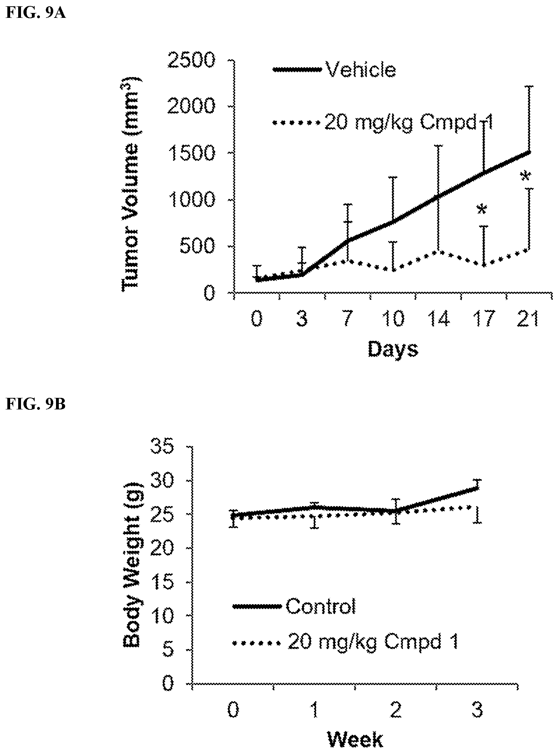

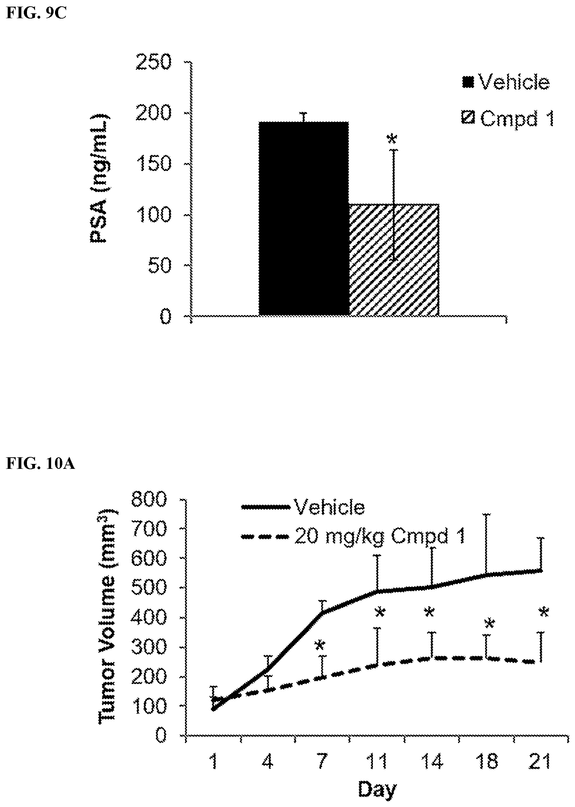

Next the effects of compound **1** *in vivo* were studied in two separate models. First, intact SCID mice were injected with resistant prostate cancer VCaP cells suspended in matrigel. Once tumors were palpable, mice were divided into two groups: control or 20 mg/kg compound **1** administered I.P. 5 days a week for 3 weeks. Tumor volumes were measured twice weekly and mouse weights were monitored. At the end of treatment, tumors and serum were collected for assessment. As seen in FIG. 9A, treatment with compound **1** significantly decreased tumor volume but had no effect on mouse body weight compared to control (FIG. 9B). Furthermore, serum PSA was significantly decreased in the group treated with compound **1** (FIG. 9C).

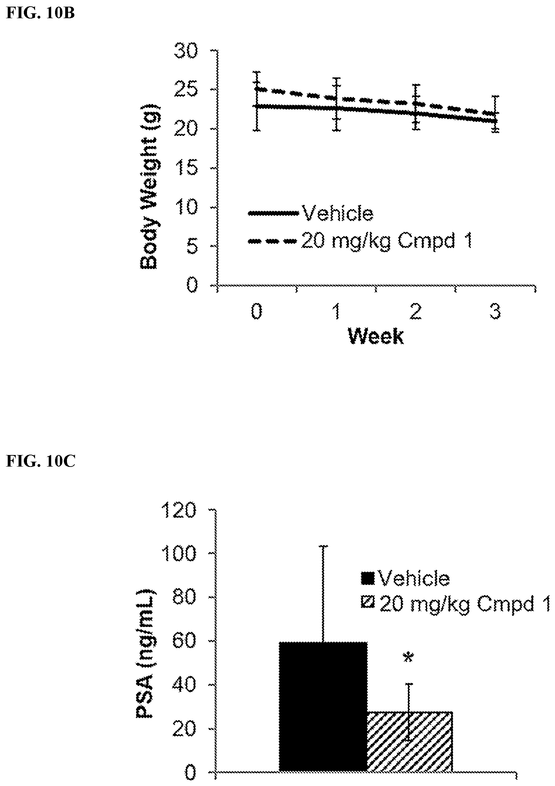

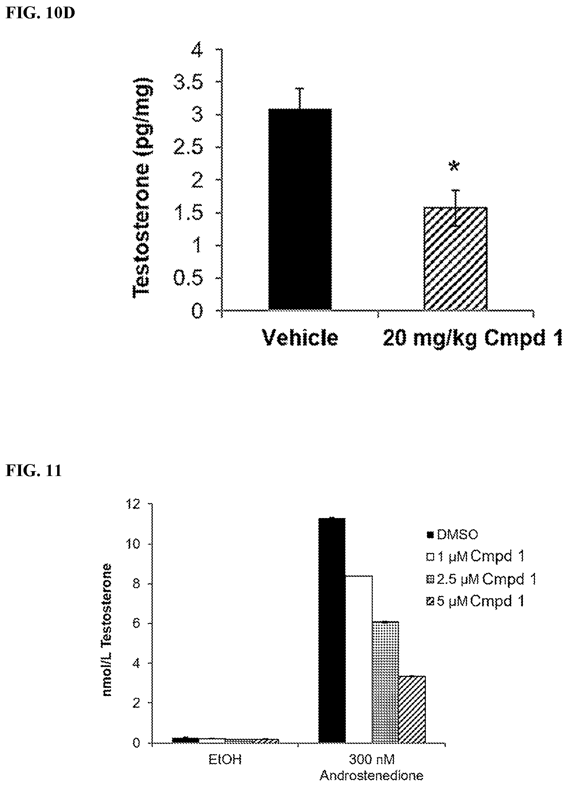

The LuCaP35CR PDX model was also employed to investigate the effects of compound **1** in vivo. For this model, SCID mice were castrated and implanted with LuCaP35CR tissues. Once tumors were palpable, mice were divided into two groups: control or 20 mg/kg compound **1** administered I.P. 5 days a week for 3 weeks. Tumor volumes were measured twice weekly and mouse weights were monitored. At the end of treatment, tumors and serum were collected for assessment. As with the VCaP model, compound **1** treatment had significantly decreased tumor volumes (FIG. 10A) but had no effect on mouse body weight (FIG. 10B). Serum PSA was also reduced by treatment with compound **1** in this model (FIG. 10C). In addition, treatment with compound **1** significantly reduced intratumoral testosterone levels FIG. 10D). These data demonstrate that compound 1 is a well-tolerated and useful for treatment of castration-resistant prostate cancer.

### Example 7. Prevention of androstenedione conversion to testosterone by AR/AKR1C3 inhibitor 1.

AKR1C3 plays a crucial role in the synthesis of testosterone, by catalyzing the conversion of the adrenal androgens dehydroepiandrosterone (DHEA) and androstenedione (AD) into testosterone. To determine the effects of compound **1** on inhibition of AKR1C3 enzymatic activity an *ex vivo* tumor based AKR1C3 enzyme assay was conducted. LUCaP35CX tumors, which express high levels of AKR1C3, were digested with 0.1% protease and cultured in medium supplemented with 10% heat-inactivated charcoal-dextran-stripped FBS. After overnight incubation, androstenedione (300 nmol/L) was added with or without compound **1.** The supernatants were collected to measure testosterone. Compound **1** showed dose-dependent inhibition of androstenedione conversion to testosterone (FIG. 11).

### Example 8. Inhibition of AR and AR-V7 signal pathways and genes associated with cancer cell proliferation and survival

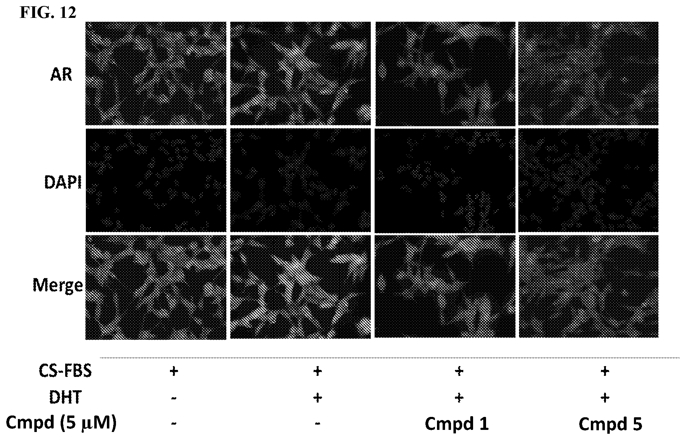

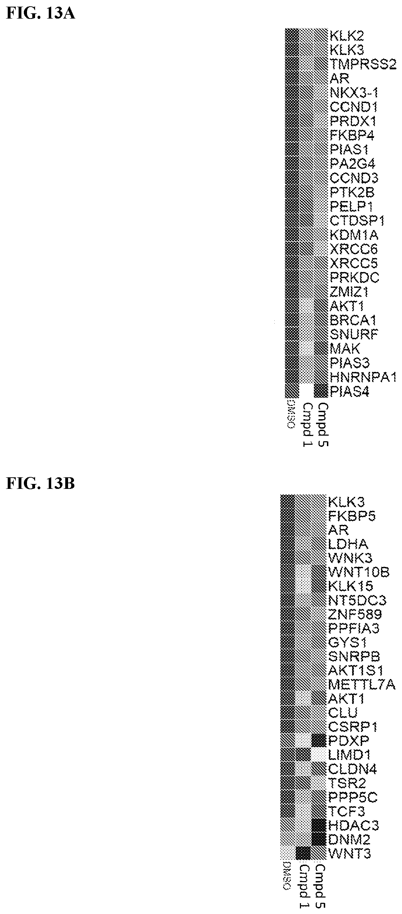

Next, the effects of the new AR/AKR1C3 inhibitors on AR signaling were studied. Compound **1** and compound **5** were found to be capable of inhibiting DHT-stimulated AR nuclear expression in LNCaP cells (FIG. 12). To further confirm the inhibitory role of the compounds in AR signaling, enzalutamide resistant C4-2B MDVR cells (which have enhanced expression of AR, AR variants, and AKR1C3) were treated with 5 µM compound **1,** 5 µM compound **5,** or a DMSO control. Total RNA was isolated assessed using RNAseq. RNAseq analysis revealed that treatment with both compounds greatly reduced expression of genes associated with AR signaling (FIG. 13A). Furthermore, these treatments also reduced expression of AR-V7 signature genes (FIG. 13B). Together, these data demonstrate that the compounds successfully inhibit the intended targets (i.e., AR/AR-V7 and AKR1C3).

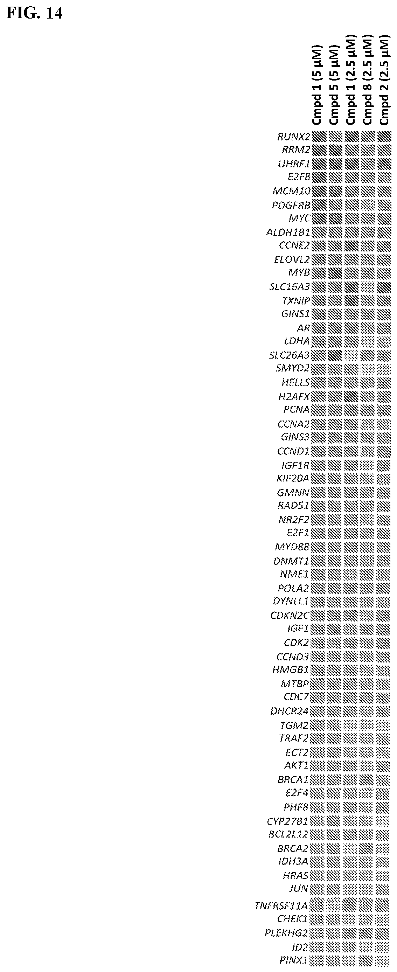

In addition, the effect of the compounds on the expression of genes involved in cancer was studied. Enzalutamide resistant C4-2B MDVR cells were treated with 5 µM of compound **1, 2, 5,** or **8,** or DMSO control. Total RNA was isolated, and RNAseq analysis revealed that treatment with the compounds greatly reduced expression of genes associated with cancer cell proliferation and survival (FIG. 14).

### VII. Exemplary Embodiments

Exemplary embodiments provided in accordance with the presently disclosed subject matter include:
1. A compound according to Formula I: 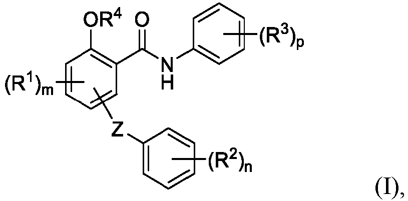 or a pharmaceutically acceptable salt thereof, wherein:
   Z is selected from the group consisting of -CH₂-, -C(O)-, -O-, -S-, and - NR^{a}-;
   Z is in the para position with respect to -OR⁴ or the ortho position with respect to -C(O)NH-;
   subscript m is 0, 1, 2, or 3
   each R¹ is independently selected from the group consisting of -OH, C₁₋₈ alkyl, C₁₋₈ alkoxy, and halogen;
   subscript n is 0, 1, 2, 3, 4, or 5;
   each R² is independently selected from the group consisting of -OH, halogen, -NO₂, -CN, C₁₋₈ alkyl, C₁₋₈ alkoxy, C₁₋₈ haloalkyl, C₂₋₈ alkenyl, C₂₋₈ alkynyl, and -N(R^{a})₂;
   subscript p is 2, 3, 4, 5, or 1;
   each R³ is independently selected from the group consisting of C₁₋₈ haloalkyl, -OH, halogen, -NO₂, -CN, C₁₋₈ alkyl, C₁₋₈ alkoxy, C₂₋₈ alkenyl, C₂₋₈ alkynyl, and -N(R^{a})₂;R⁴ is selected from the group consisting of H, α-aminoacyl; -S(O)₂N(R^{a})₂, -C(O)N(R^{a})₂, and -C(O)R^{b};
   each R^{a} is independently selected from the group consisting of H and C₁₋₈ alkyl;
   R^{b} is C₁₋₈ alkyl;
   provided that:
      if Z is -CH₂- in the para position with respect to -OR⁴, subscript m and subscript n are 0, and R⁴ is H, then at least one R³ is selected from the group consisting of halogen, -NO₂, C₂₋₈ alkyl, C₁₋₈ alkoxy, C₁₋₈ haloalkyl, C₂₋₈ alkenyl, C₂₋₈ alkynyl, and -N(R^{a})₂;
      if Z is -CH₂- in the para position with respect to -OR⁴ and the compound is a 2,3,4-trihydroxy-benzamide, then at least one R² or at least one R³ is selected from the group consisting of -OH,-NO₂, -CN, C₁₋₈ alkoxy, C₁₋₈ haloalkyl, C₂₋₈ alkenyl, C₂₋₈ alkynyl, and -N(R^{a})₂;
      if Z is -CH₂- in the para position with respect to -OR⁴ and the compound is a 5-alkyl-6-hydroxy-benzamide, then at least one R² or at least one R³ is selected from the group consisting of -OH,-NO₂, -CN, C₁₋₈ alkyl, C₁₋₈ alkoxy, C₁₋₈ haloalkyl, C₂₋₈ alkenyl, C₂₋₈ alkynyl, and -N(R^{a})₂;
      if Z is -C(O)- in the para position with respect to -OR⁴ and the compound is a 2-hydroxy-benzamide, a 2,5-dialkyl-6-hydroxy-benzamide, or a 5-alkyl-6-hydroxy-benzamide, then (i) at least one R² or at least one R³ is selected from the group consisting of - OH, C₁₋₈ alkoxy, C₁₋₈ haloalkyl, C₂₋₈ alkenyl, C₂₋₈ alkynyl, and -N(R^{a})₂, and (ii) at least one R³ is other than -CF₃ or -NO₂ when subscript n is 1 and R² is 4-chloro or 4-(n-butyl); and
      if Z is -S- in the para position with respect to -OR⁴ and the compound is a 2,5-dialkyl-6-hydroxy-benzamide or a 5-alkyl-6-hydroxy-benzamide, then at least one R² or at least one R³ is selected from the group consisting of -OH, C₂₋₈ alkyl, C₂₋₈ alkoxy, C₂₋₈ alkenyl, C₂₋₈ alkynyl, and -N(R^{a})₂.
2. The compound of embodiment 1, or a pharmaceutically acceptable salt thereof, wherein Z is -CH₂-.
3. The compound of embodiment 1, or a pharmaceutically acceptable salt thereof, wherein Z is -C(O)-.
4. The compound of embodiment 1, or a pharmaceutically acceptable salt thereof, wherein Z is -O-.
5. The compound of any one of embodiments 1-4, or a pharmaceutically acceptable salt thereof, wherein subscript m is 0.
6. The compound of any one of embodiments 1-5, or a pharmaceutically acceptable salt thereof, wherein each R³ is independently selected from the group consisting of -OH, halogen, -NO₂, -CN, C₁₋₈ alkyl, C₁₋₈ alkoxy, C₁₋₈ haloalkyl, C₂₋₈ alkenyl, and C₂₋₈ alkynyl.
7. The compound of any one of embodiments 1-5, or a pharmaceutically acceptable salt thereof, wherein each R³ is independently selected from the group consisting of C₁₋₈ haloalkyl, halogen, and -NO₂.
8. The compound of any one of embodiments 1-7, or a pharmaceutically acceptable salt thereof, wherein subscript p is 1 or 2.
9. The compound of embodiment 8, having a structure according to Formula IIa: 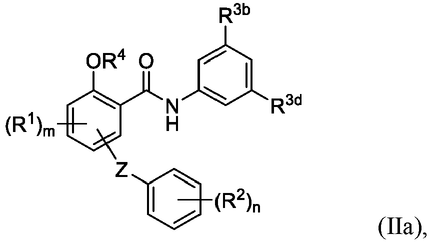 or a pharmaceutically acceptable salt thereof.
10. The compound of embodiment 9, or a pharmaceutically acceptable salt thereof, wherein R^{3b} and R^{3d} are independently C₁₋₈ haloalkyl.
11. The compound of embodiment 10, or a pharmaceutically acceptable salt thereof, wherein R^{3b} and R^{3d} are independently selected from the group consisting of - CF₃ and -CCl₃.
12. The compound of embodiment 8, having a structure according to Formula IIb: 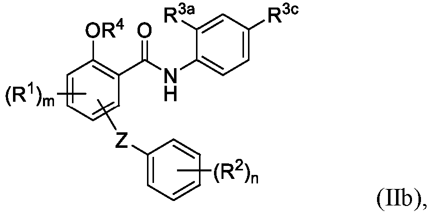 or a pharmaceutically acceptable salt thereof.
13. The compound of embodiment 12, or a pharmaceutically acceptable salt thereof, wherein R^{3a} and R^{3c} are independently selected from the group consisting of halogen and -NO₂.
14. The compound of embodiment 13, or a pharmaceutically acceptable salt thereof, wherein R^{3a} is -Cl and R^{3c} is -NO₂.
15. The compound of embodiment 8 having a structure according to Formula IIc: 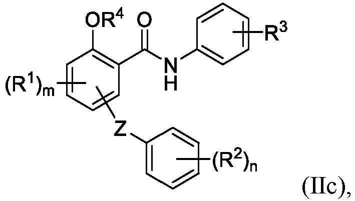 or a pharmaceutically acceptable salt thereof.
16. The compound of embodiment 15, or a pharmaceutically acceptable salt thereof, wherein R³ is C₁₋₈ haloalkyl.
17. The compound of embodiment 15, or a pharmaceutically acceptable salt thereof, wherein R³ is selected from the group consisting of -CF₃ and -CCl₃.
18. The compound of any one of embodiments 1-17, or a pharmaceutically acceptable salt thereof, wherein subscript n is 0.
19. The compound of any one of embodiments 1-17, or a pharmaceutically acceptable salt thereof, wherein subscript n is 1.
20. The compound of embodiment 19, or a pharmaceutically acceptable salt thereof, wherein R² is selected from the group consisting of C₁₋₈ alkyl, C₁₋₈ alkoxy, and C₁₋₈ haloalkyl
21. The compound of embodiment 19 or embodiment 20, having a structure according to Formula IIIa: 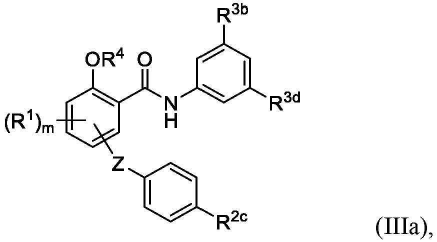 or a pharmaceutically acceptable salt thereof.
22. The compound of embodiment 21, or a pharmaceutically acceptable salt thereof, wherein R^{3b} and R^{3d} are independently C₁₋₈ haloalkyl.
23. The compound of embodiment 22, or a pharmaceutically acceptable salt thereof, wherein R^{3b} and R^{3d} are independently selected from the group consisting of - CF₃ and -CCl₃.
24. The compound of embodiment 19 or embodiment 20, having a structure according to Formula IIIb: 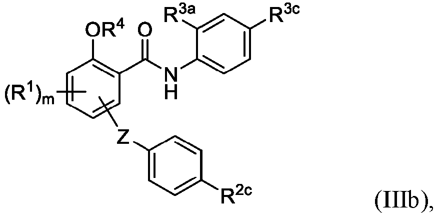 or a pharmaceutically acceptable salt thereof.
25. The compound of embodiment 24, or a pharmaceutically acceptable salt thereof, wherein R^{3a} and R^{3c} are independently selected from the group consisting of halogen and -NO₂.
26. The compound of embodiment 25, or a pharmaceutically acceptable salt thereof, wherein R^{3a} is -Cl and R^{3c} is -NO₂.
27. The compound of embodiment 19 or embodiment 20, having a structure according to Formula IIIc: 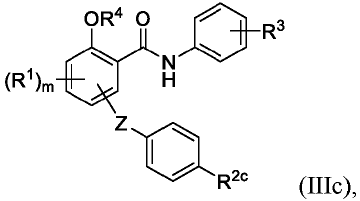 or a pharmaceutically acceptable salt thereof.
28. The compound of embodiment 27, or a pharmaceutically acceptable salt thereof, wherein R³ is C₁₋₈ haloalkyl.
29. The compound of embodiment 28, or a pharmaceutically acceptable salt thereof, wherein R³ is selected from the group consisting of -CF₃ and -CCl₃.
30. The compound of any one of embodiments 21-29, or a pharmaceutically acceptable salt thereof, wherein R^{2c} is -OCH₃.
31. The compound of any one of embodiments 1-30, or a pharmaceutically acceptable salt thereof, wherein R⁴ is H or α-aminoacyl.
32. The compound of embodiment 31, or a pharmaceutically acceptable salt thereof, wherein R⁴ is selected from the group consisting of l-valinyl and d-valinyl.
33. The compound of any one of embodiments 1-32, or a pharmaceutically acceptable salt thereof, wherein Z is present in the para position with respect to -OR⁴.
34. The compound of any one of embodiments 1-32, or a pharmaceutically acceptable salt thereof, wherein Z is present in the ortho position with respect to -C(O)NH-.
35. The compound of embodiment 1, which is selected from the group consisting of: 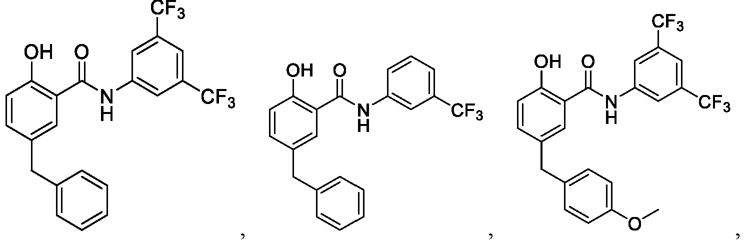 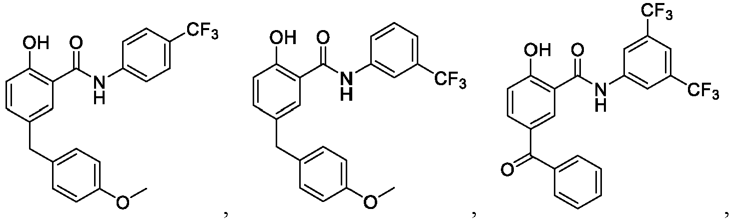 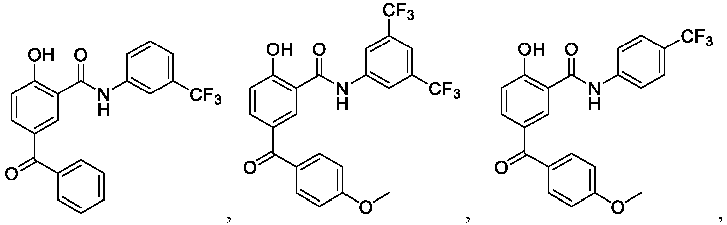 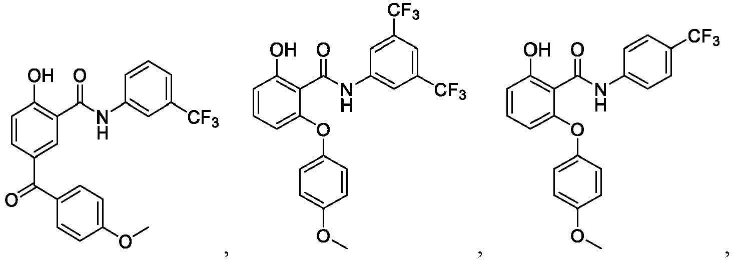 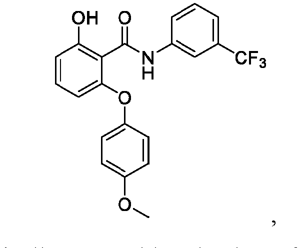 and pharmaceutically acceptable salts thereof.
36. The compound of embodiment 1, or a pharmaceutically acceptable salt thereof, wherein:
   Z is -CH₂-;
   subscript m and subscript n are 0; and
   at least one R³ is selected from the group consisting of halogen, -NO₂, C₁₋₈ alkoxy, C₁₋₈ haloalkyl, C₂₋₈ alkenyl, C₂₋₈ alkynyl, and -N(R^{a})₂.
37. The compound of embodiment 1, or a pharmaceutically acceptable salt thereof, wherein:
   Z is -C(O)- in the para position with respect to -OR⁴;
   the compound is a 2-hydroxy-benzamide, a 2,5-dialkyl-6-hydroxy-benzamide, or a 5-alkyl-6-hydroxy-benzamide; and
   at least one R² or at least one R³ is selected from the group consisting of -OH, C₁₋₈ alkoxy, C₂₋₈ alkenyl, C₂₋₈ alkynyl, and -N(R^{a})₂.
38. The compound of embodiment 1, or a pharmaceutically acceptable salt thereof, wherein:
   Z is -S- in the para position with respect to -OR⁴;
   the compound is a 2,5-dialkyl-6-hydroxy-benzamide or a 5-alkyl-6-hydroxy-benzamide; and
   at least one R² or at least one R³ is selected from the group consisting of -OH, C₂₋₈ alkenyl, C₂₋₈ alkynyl, and -N(R^{a})₂.
39. A pharmaceutical composition comprising a compound according to any one of embodiments 1-38, or a pharmaceutically acceptable salt thereof, and a pharmaceutically acceptable excipient.
40. A compound according to any one of embodiments 1-38 or a pharmaceutically acceptable salt thereof, or a pharmaceutical composition according to embodiment 39 for use in a method for treating a hormone-mediated disease or condition, the method comprising administering to a subject in need thereof a therapeutically effective amount of the compound according to any one of embodiments 1-38 or a pharmaceutically acceptable salt thereof, or a therapeutically effective amount of the pharmaceutical composition according to embodiment 39, thereby treating the hormone-mediated disease or condition.
41. The compound or pharmaceutical composition for use of embodiment 40, wherein the hormone-mediated disease is a cancer.
42. The compound or pharmaceutical composition for use of embodiment 41, wherein the cancer is castration resistant prostate cancer.
43. The compound or pharmaceutical composition for use of any one of embodiments 40-42, further comprising administering an antiandrogen agent to the subject.
44. The compound or pharmaceutical composition for use of embodiment 43, wherein the antiandrogen agent is selected from the group consisting of enzalutamide, apalutamide, darolutamide, abiraterone, pharmaceutically acceptable salts thereof, and combinations thereof.

Also disclosed is a method for inhibiting an androgen receptor (AR), the method comprising contacting the AR with an effective amount of a compound according to any one of embodiments 1-38 or a salt thereof, thereby inhibiting the AR.

Also disclosed is a method for inhibiting aldo-keto reductase family 1 member C3 (AKR1C3), the method comprising contacting the AKR1C3 with an effective amount of a compound according to any one of embodiments 1-38 or a salt thereof, thereby inhibiting the AKR1C3.

## Claims

1. A compound according to Formula I: 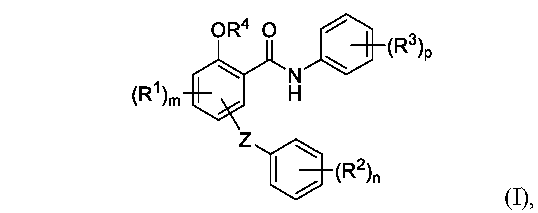 or a pharmaceutically acceptable salt thereof, wherein:
Z is selected from the group consisting of -CH₂-, -C(O)-, -O-, -S-, and - NR^{a}-;
Z is in the para position with respect to -OR⁴ or the ortho position with respect to -C(O)NH-;
subscript m is 0, 1, 2, or 3;
each R¹ is independently selected from the group consisting of -OH, C₁₋₈ alkyl, C₁₋₈ alkoxy, and halogen;
subscript n is 0, 1, 2, 3, 4, or 5;
each R² is independently selected from the group consisting of -OH, halogen, -NO₂, -CN, C₁₋₈ alkyl, C₁₋₈ alkoxy, C₁₋₈ haloalkyl, C₂₋₈ alkenyl, C₂₋₈ alkynyl, and -N(R^{a})₂;
subscript p is 2, 3, 4, 5, or 1;
each R³ is independently selected from the group consisting of C₁₋₈ haloalkyl, -OH, halogen, -NO₂, -CN, C₁₋₈ alkyl, C₁₋₈ alkoxy, C₂₋₈ alkenyl, C₂₋₈ alkynyl, and -N(R^{a})₂;
R⁴ is selected from the group consisting of H, α-aminoacyl; -S(O)₂N(R^{a})₂, -C(O)N(R^{a})₂, and -C(O)R^{b};
each R^{a} is independently selected from the group consisting of H and C₁₋₈ alkyl; and
R^{b} is C₁₋₈ alkyl;
provided that:
if Z is -CH₂- in the para position with respect to -OR⁴, subscript m and subscript n are 0, and R⁴ is H, then at least one R³ is selected from the group consisting of C₁₋₈ haloalkyl, halogen, -NO₂, C₂₋₈ alkyl, C₁₋₈ alkoxy, C₂₋₈ alkenyl, C₂₋₈ alkynyl, and -N(R^{a})₂;
if Z is -CH₂- in the para position with respect to -OR⁴ and the compound is a 2,3,4-trihydroxy-benzamide, then at least one R² or at least one R³ is selected from the group consisting of -OH,-NO₂, -CN, C₁₋₈ alkoxy, C₁₋₈ haloalkyl, C₂₋₈ alkenyl, C₂₋₈ alkynyl, and -N(R^{a})₂;
if Z is -CH₂- in the para position with respect to -OR⁴ and the compound is a 5-alkyl-6-hydroxy-benzamide, then at least one R² or at least one R³ is selected from the group consisting of -OH,-NO₂, -CN, C₁₋₈ alkyl, C₁₋₈ alkoxy, C₁₋₈ haloalkyl, C₂₋₈ alkenyl, C₂₋₈ alkynyl, and -N(R^{a})₂;
if Z is -C(O)- in the para position with respect to -OR⁴ and the compound is a 2-hydroxy-benzamide, a 2,5-dialkyl-6-hydroxy-benzamide, or a 5-alkyl-6-hydroxy-benzamide, then (i) at least one R² or at least one R³ is selected from the group consisting of - OH, C₁₋₈ alkoxy, C₁₋₈ haloalkyl, C₂₋₈ alkenyl, C₂₋₈ alkynyl, and -N(R^{a})₂, and (ii) at least one R³ is other than -CF₃ or -NO₂ when subscript n is 1 and R² is 4-chloro or 4-(n-butyl); and
if Z is -S- in the para position with respect to -OR⁴ and the compound is a 2,5-dialkyl-6-hydroxy-benzamide or a 5-alkyl-6-hydroxy-benzamide, then at least one R² or at least one R³ is selected from the group consisting of -OH, C₂₋₈ alkyl, C₂₋₈ alkoxy, C₂₋₈ alkenyl, C₂₋₈ alkynyl, and -N(R^{a})₂.

2. The compound of claim 1, or a pharmaceutically acceptable salt thereof, wherein:
(A) Z is -CH₂-, -C(O)-, or -O-; and/or
(B) subscript m is 0; and/or
(C) each R³ is independently selected from the group consisting of (i) C₁₋₈ haloalkyl, -OH, halogen, -NO₂, -CN, C₁₋₈ alkyl, C₁₋₈ alkoxy, C₂₋₈ alkenyl, and C₂₋₈ alkynyl, or (ii) C₁₋₈ haloalkyl, halogen, and -NO₂.

3. The compound of claim 1 or 2, or a pharmaceutically acceptable salt thereof, wherein subscript p is 1 or 2.

4. The compound of claim 3, having a structure:
(A) according to Formula IIa: 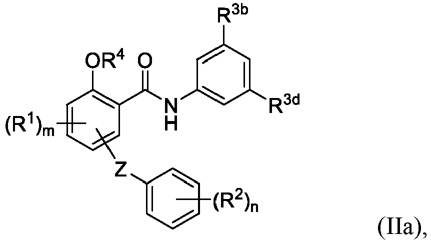 or a pharmaceutically acceptable salt thereof, optionally wherein R^{3b} and R^{3d} are (i) independently C₁₋₈ haloalkyl or (ii) independently selected from the group consisting of -CF₃ and -CCl₃; or
(B) according to Formula IIb: 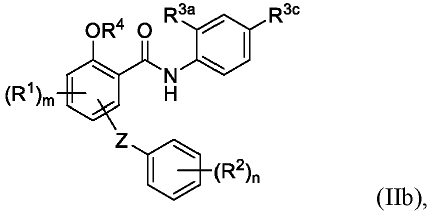 or a pharmaceutically acceptable salt thereof, optionally wherein (i) R^{3a} and R^{3c} are independently selected from the group consisting of halogen and -NO₂ or (ii) R^{3a} is -Cl and R^{3c} is -NO₂; or
(C) according to Formula IIc: 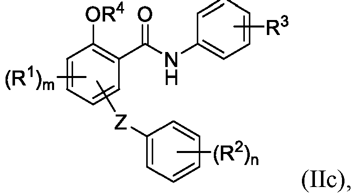 or a pharmaceutically acceptable salt thereof, optionally wherein R³ is (i) C₁₋₈ haloalkyl or (ii) selected from the group consisting of -CF₃ and -CCl₃.

5. The compound of any one of claims 1 to 4, or a pharmaceutically acceptable salt thereof, wherein subscript n is 0.

6. The compound of any one of claims 1 to 4, or a pharmaceutically acceptable salt thereof, wherein subscript n is 1, and optionally wherein R² is selected from the group consisting of C₁₋₈ alkyl, C₁₋₈ alkoxy, and C₁₋₈ haloalkyl

7. The compound of claim 6, having a structure:
(A) according to Formula IIIa: 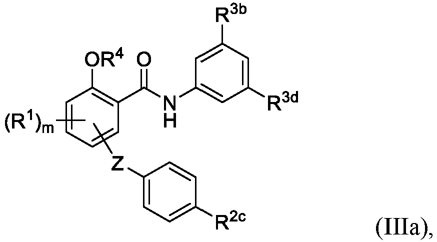 or a pharmaceutically acceptable salt thereof, optionally wherein R^{3b} and R^{3d} are (i) independently C₁₋₈ haloalkyl or (ii) independently selected from the group consisting of -CF₃ and -CCl₃; or
(B) according to Formula IIIb: 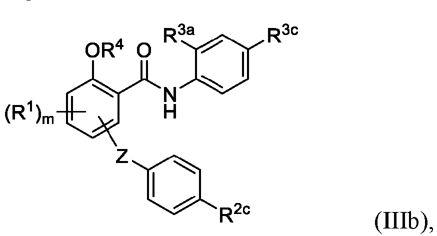 or a pharmaceutically acceptable salt thereof, optionally wherein (i) R^{3a} and R^{3c} are independently selected from the group consisting of halogen and -NO₂ or (ii) R^{3a} is -Cl and R^{3c} is -NO₂; or
(C) according to Formula IIIc: 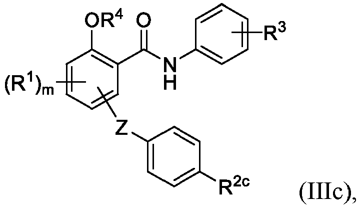 or a pharmaceutically acceptable salt thereof, optionally wherein R³ is (i) C₁₋₈ haloalkyl or (ii) selected from the group consisting of -CF₃ and -CCl₃.

8. The compound of claim 7, or a pharmaceutically acceptable salt thereof, wherein R^{2c} is -OCH₃.

9. The compound of any one of claims 1 to 8, or a pharmaceutically acceptable salt thereof, wherein R⁴ is (i) H or α-aminoacyl or (ii) selected from the group consisting of l-valinyl and d-valinyl.

10. The compound of claim 1, which is selected from the group consisting of: 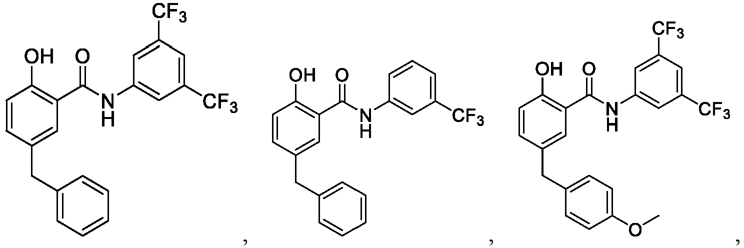 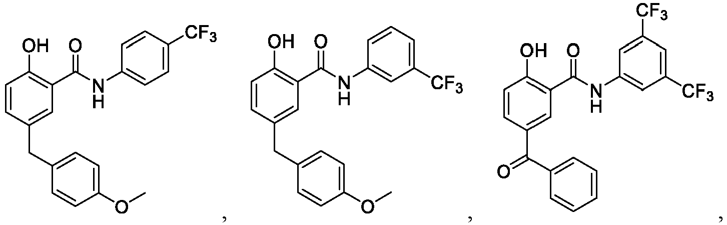 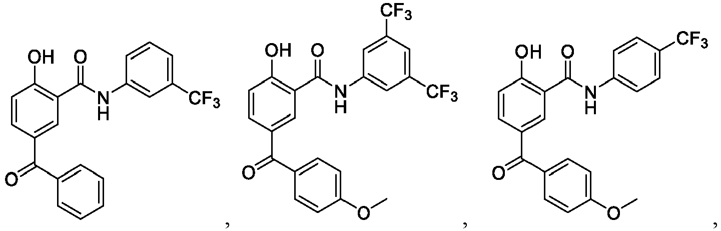 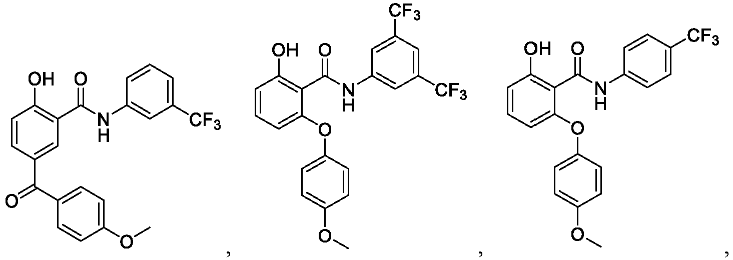 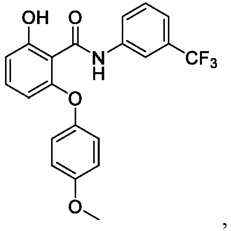 and pharmaceutically acceptable salts thereof.

11. The compound of claim 1, or a pharmaceutically acceptable salt thereof, wherein:
(A) Z is -CH₂-; subscript m and subscript n are 0; and at least one R³ is selected from the group consisting of halogen, -NO₂, C₁₋₈ alkoxy, C₁₋₈ haloalkyl, C₂₋₈ alkenyl, C₂₋₈ alkynyl, and -N(R^{a})₂; or
(B) Z is -C(O)- in the para position with respect to -OR⁴; the compound is a 2-hydroxy-benzamide, a 2,5-dialkyl-6-hydroxy-benzamide, or a 5-alkyl-6-hydroxy-benzamide; and at least one R² or at least one R³ is selected from the group consisting of -OH, C₁₋₈ alkoxy, C₂₋₈ alkenyl, C₂₋₈ alkynyl, and -N(R^{a})₂; or
(C) Z is -S- in the para position with respect to -OR⁴; the compound is a 2,5-dialkyl-6-hydroxy-benzamide or a 5-alkyl-6-hydroxy-benzamide; and at least one R² or at least one R³ is selected from the group consisting of -OH, C₂₋₈ alkenyl, C₂₋₈ alkynyl, and -N(R^{a})₂.

12. A pharmaceutical composition comprising a compound according to any one of claims 1-11, or a pharmaceutically acceptable salt thereof, and a pharmaceutically acceptable excipient.

13. A compound according to any one of claims 1 to 11, or a pharmaceutical composition according to claim 12, for use as a medicament.

14. A compound according to any one of claims 1 to 11, or a pharmaceutical composition according to claim 12, for use in a method for treating a hormone-mediated disease or condition, the method comprising administering to a subject in need thereof a therapeutically effective amount of the compound or pharmaceutical composition, thereby treating the hormone-mediated disease or condition, optionally wherein the hormone-mediated disease is a cancer, optionally wherein the cancer is castration resistant prostate cancer.

15. The compound or pharmaceutical composition for use of claim 14, the method further comprising administering an antiandrogen agent to the subject, optionally wherein the antiandrogen agent is selected from the group consisting of enzalutamide, apalutamide, darolutamide, abiraterone, pharmaceutically acceptable salts thereof, and combinations thereof.

## Patentansprüche

1. Verbindung nach Formel I: 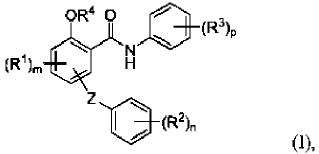 oder ein pharmazeutisch annehmbares Salz davon, wobei:
Z ausgewählt ist aus der Gruppe bestehend aus -CF₂-, -C(O)-, -O-, -S- und -NR^{a}-;
Z sich in para-Stellung in Bezug auf -OR⁴ oder in ortho-Stellung in Bezug auf - C(O)NH- befindet;
das tiefgestellte m 0, 1, 2 oder 3 ist;
jedes R¹ unabhängig ausgewählt ist aus der Gruppe bestehend aus -OH, C₁₋₈-Alkyl, C₁₋₈-Alkoxy und Halogen;
das tiefgestellte n 0, 1, 2, 3, 4 oder 5 ist;
jedes R² unabhängig ausgewählt ist aus der Gruppe bestehend aus -OH, Halogen, - NO₂, -CN, C₁₋₈-Alkyl, C₁₋₈-Alkoxy, C₁₋₈-Halogenalkyl, C₂₋₈-Alkenyl, C₂₋₈-Alkinyl und -N(R^{a})₂;
das tiefgestellte p 2, 3, 4, 5 oder 1 ist;
jedes R³ unabhängig ausgewählt ist aus der Gruppe bestehend aus C₁₋₈-Halogenalkyl, OH, Halogen, -NO₂, -CN, C₁₋₈-Alkyl, C₁₋₈-Alkoxy, C₂₋₈-Alkenyl, C₂₋₈-Alkinyl und -N(R^{a})₂;
R⁴ ausgewählt ist aus der Gruppe bestehend aus H, α-Aminoacyl; -S(O)₂N(R^{a})₂, C(O)N(R^{a})₂ und -C(O)R^{b};
jedes R^{a} unabhängig ausgewählt ist aus der Gruppe bestehend aus H und C₁₋₈-Alkyl; und
R^{b} C₁₋₈-Alkyl ist;
mit der Maßgabe, dass:
wenn Z in para-Stellung in Bezug auf -OR⁴ -CH₂- ist, das tiefgestellte m und das tiefgestellte n 0 sind und R⁴ H ist, dann mindestens ein R³ ausgewählt ist aus der Gruppe bestehend aus C₁₋₈-Halogenalkyl, Halogen, -NO₂, C₂₋₈-Alkyl, C₁₋₈-Alkoxy, C₂₋₈-Alkenyl, C₂₋₈-Alkinyl und -N(Ra)₂;
wenn Z in para-Stellung in Bezug auf -OR⁴ -CH₂- ist und die Verbindung ein 2,3,4-Trihydroxybenzamid ist, mindestens ein R² oder mindestens ein R³ ausgewählt ist aus der Gruppe bestehend aus -OH, -NO₂, -CN, C₁₋₈-Alkoxy, C₁₋₈-Halogenalkyl, C₂₋₈-Alkenyl, C₂₋₈-Alkinyl und -N(R^{a})₂;
wenn Z in para-Stellung in Bezug auf -OR₄ -CH₂- ist und die Verbindung ein 5-Alkyl-6-hydroxybenzamid ist, mindestens ein R² oder mindestens ein R³ ausgewählt ist aus der Gruppe bestehend aus -OH, -NO₂, -CN, C₁₋₈-Alkyl, C₁₋₈-Alkoxy, C₁₋₈-Halogenalkyl, C₂₋₈-Alkenyl, C₂₋₈-Alkinyl und -N(R^{a})₂;
wenn Z in para-Stellung in Bezug auf -OR⁴ -C(O)- ist und die Verbindung ein 2-Hydroxybenzamid, ein 2,5-Dialkyl-6-hydroxybenzamid oder ein 5-Alkyl-6-hydroxybenzamid ist, (i) mindestens ein R² oder mindestens ein R³ ausgewählt ist aus der Gruppe bestehend aus -OH, C₁₋₈-Alkoxy, C₁₋₈-Halogenalkyl, C₂₋₈-Alkenyl, C₂₋₈-Alkinyl und -N(R^{a})₂, und (ii) mindestens ein R³ von -CF₃ oder -NO₂ verschieden ist, wenn das tiefgestellte n 1 ist und R² 4-Chlor oder 4-(*n*-Butyl) ist; und
wenn Z in para-Stellung in Bezug auf -OR⁴ -S- ist und die Verbindung ein 2,5-Dialkyl-6-hydroxybenzamid oder ein 5-Alkyl-6-hydroxybenzamid ist, mindestens ein R² oder mindestens ein R³ ausgewählt ist aus der Gruppe bestehend aus -OH, C₂₋₈-Alkyl, C₂₋₈-Alkoxy, C₂₋₈-Alkenyl, C₂₋₈-Alkinyl und -N(R^{a})₂.

2. Verbindung nach Anspruch 1 oder ein pharmazeutisch annehmbares Salz davon, wobei:
(A) Z -CH₂-, -C(O)- oder -O- ist; und/oder
(B) das tiefgestellte m 0 ist; und/oder
(C) jedes R³ unabhängig ausgewählt ist aus der Gruppe bestehend aus (i) C₁₋₈-Halogenalkyl, -OH, Halogen, -NO₂, -CN, C₁₋₈-Alkyl, C₁₋₈-Alkoxy, C₂₋₈-Alkenyl und C₂₋₈-Alkinyl oder (ii) C₁₋₈-Halogenalkyl, Halogen und -NO₂.

3. Verbindung nach Anspruch 1 oder 2 oder ein pharmazeutisch annehmbares Salz davon, wobei das tiefgestellte p 1 oder 2 ist.

4. Verbindung nach Anspruch 3 mit einer Struktur:
(A) gemäß Formel IIa: 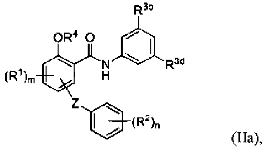 oder ein pharmazeutisch annehmbares Salz davon, wobei R^{3b} und R^{3d} optional (i) unabhängig voneinander C₁₋₈-Halogenalkyl sind oder (ii) unabhängig voneinander ausgewählt sind aus der Gruppe bestehend aus -CF₃ und -CCl₃; oder
(B) gemäß Formel IIb: 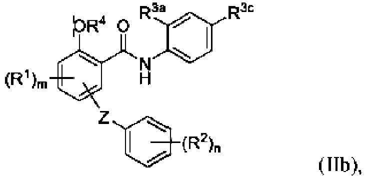 oder ein pharmazeutisch annehmbares Salz davon, wobei (i) R^{3a} und R^{3c} optional unabhängig voneinander ausgewählt sind aus der Gruppe bestehend aus Halogen und -NO₂, oder (ii) R^{3a} -Cl ist und R^{3c} -NO₂ ist; oder
(C) gemäß Formel IIc: 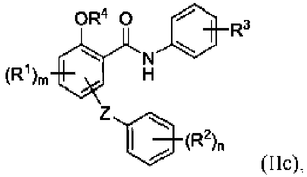 oder ein pharmazeutisch annehmbares Salz davon, wobei R³ optional (i) C₁₋₈-Halogenalkyl ist oder (ii) ausgewählt ist aus der Gruppe bestehend aus -CF₃ und -CCl₃.

5. Verbindung nach einem der Ansprüche 1 bis 4 oder ein pharmazeutisch annehmbares Salz davon, wobei das tiefgestellte n 0 ist.

6. Verbindung nach einem der Ansprüche 1 bis 4 oder ein pharmazeutisch annehmbares Salz davon, wobei das tiefgestellte n 1 ist und wobei R² optional ausgewählt ist aus der Gruppe bestehend aus C₁₋₈-Alkyl, C₁₋₈-Alkoxy und C₁₋₈-Halogenalkyl.

7. Verbindung nach Anspruch 6 mit einer Struktur:
(A) gemäß Formel IIIa: 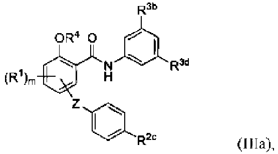 oder ein pharmazeutisch annehmbares Salz davon, wobei R^{3b} und R^{3d} optional (i) unabhängig voneinander C₁₋₈-Halogenalkyl sind oder (ii) unabhängig voneinander ausgewählt sind aus der Gruppe bestehend aus -CF₃ und -CCl₃; oder
(B) gemäß Formel IIIb: 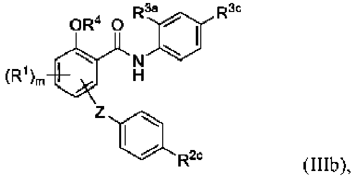 oder ein pharmazeutisch annehmbares Salz davon, wobei (i) R^{3a} und R^{3c} optional unabhängig voneinander ausgewählt sind aus der Gruppe bestehend aus Halogen und -NO₂ oder (ii) R^{3a} -Cl ist und R^{3c} -NO₂ ist; oder
(C) gemäß Formel IIIc: 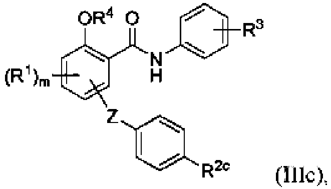 oder ein pharmazeutisch annehmbares Salz davon, wobei R³ optional (i) C₁₋₈-Halogenalkyl ist oder (ii) ausgewählt ist aus der Gruppe bestehend aus -CF₃ und -CCl₃.

8. Verbindung nach Anspruch 7 oder ein pharmazeutisch annehmbares Salz davon, wobei R^{2c} -OCH₃ ist.

9. Verbindung nach einem der Ansprüche 1 bis 8 oder ein pharmazeutisch annehmbares Salz davon, wobei R⁴ (i) H oder α-Aminoacyl ist oder (ii) aus der Gruppe ausgewählt ist, die aus 1-Valinyl und d-Valinyl besteht.

10. Verbindung nach Anspruch 1, ausgewählt aus der Gruppe bestehend aus: 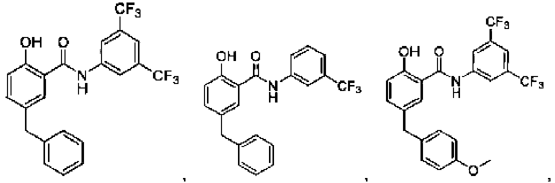 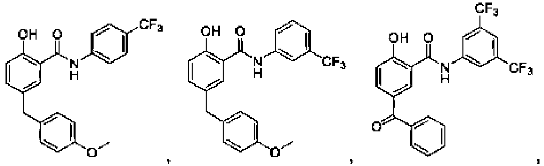 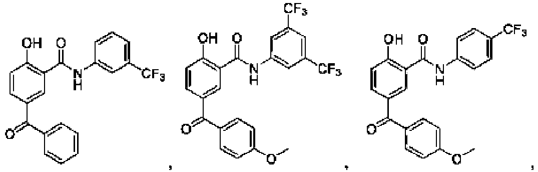 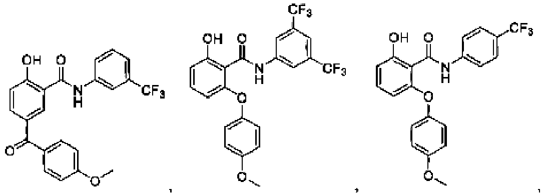 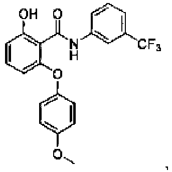 und pharmazeutisch annehmbare Salze davon.

11. Verbindung nach Anspruch 1 oder ein pharmazeutisch annehmbares Salz davon, wobei:
(A) Z -CH₂- ist; das tiefgestellte m und das tiefgestellte n 0 sind; und mindestens ein R³ ausgewählt ist aus der Gruppe bestehend aus Halogen, -NO₂, C₁₋₈-Alkoxy, C₁₋₈-Halogenalkyl, C₂₋₈-Alkenyl, C₂₋₈-Alkinyl und -N(R^{a})₂; oder
(B) Z in para-Stellung in Bezug auf -OR⁴ -C(O)- ist; die Verbindung ein 2-Hydroxybenzamid, ein 2,5-Dialkyl-6-hydroxybenzamid oder ein 5-Alkyl-6-hydroxybenzamid ist; und mindestens ein R² oder mindestens ein R³ ist ausgewählt aus der Gruppe bestehend aus -OH, C₁₋₈-Alkoxy, C₂₋₈-Alkenyl, C₂₋₈-Alkinyl und -N(R^{a})₂; oder
(C) Z in para-Stellung in Bezug auf -OR⁴ -S- ist; die Verbindung ein 2,5-Dialkyl-6-hydroxybenzamid oder ein 5-Alkyl-6-hydroxybenzamid ist; und mindestens ein R² oder mindestens ein R³ ausgewählt ist aus der Gruppe bestehend aus -OH, C₂₋₈-Alkenyl, C₂₋₈-Alkinyl und -N(R^{a})₂.

12. Pharmazeutische Zusammensetzung, umfassend eine Verbindung nach einem der Ansprüche 1 bis 11 oder ein pharmazeutisch annehmbares Salz davon sowie einen pharmazeutisch annehmbaren Hilfsstoff.

13. Verbindung nach einem der Ansprüche 1 bis 11 oder eine pharmazeutische Zusammensetzung nach Anspruch 12 zur Verwendung als Arzneimittel.

14. Verbindung nach einem der Ansprüche 1 bis 11 oder eine pharmazeutische Zusammensetzung nach Anspruch 12 zur Verwendung in einem Verfahren zur Behandlung einer hormonvermittelten Erkrankung oder eines hormonvermittelten Zustands, wobei das Verfahren die Verabreichung einer therapeutisch wirksamen Menge der Verbindung oder der pharmazeutischen Zusammensetzung an ein Subjekt, das dieser bedarf, umfasst, wodurch die hormonvermittelte Erkrankung oder der hormonvermittelte Zustand behandelt wird, wobei die hormonvermittelte Erkrankung optional eine Krebserkrankung ist, wobei die Krebserkrankung optional ein kastrationsresistenter Prostatakrebs ist.

15. Verbindung oder pharmazeutische Zusammensetzung zur Verwendung nach Anspruch 14, wobei das Verfahren ferner die Verabreichung eines antiandrogenen Mittels an das Subjekt umfasst, wobei das antiandrogene Mittel optional ausgewählt ist aus der Gruppe bestehend aus Enzalutamid, Apalutamid, Darolutamid, Abirateron, pharmazeutisch verträglichen Salzen davon und Kombinationen davon.

## Revendications

1. Composé selon la formule I : 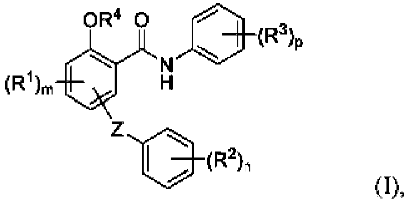 ou sel pharmaceutiquement acceptable de celui-ci, dans lequel :
Z est sélectionné dans le groupe constitué de : -CH₂-, -C(O)- , -O-, -S- et -NR^{a}-,
Z est en position para par rapport à -OR⁴ ou en position ortho par rapport à -C(O)NH-,
l'indice m vaut 0, 1, 2 ou 3,
chaque R¹ est sélectionné indépendamment dans le groupe constitué de : -OH, alkyle en C₁₋₈, alcoxy en C₁₋₈ et halogène,
l'indice n vaut 0, 1, 2, 3, 4 ou 5,
chaque R² est sélectionné indépendamment dans le groupe constitué de : -OH, halogène, -NO₂, -CN, alkyle en C₁₋₈, alcoxy en C₁₋₈, halogénoalkyle en C₁₋₈, alcényle en C₂₋₈, alcynyle en C₂₋₈ et -N(R^{a})₂,
l'indice p vaut 2, 3, 4, 5 ou 1,
chaque R³ est sélectionné indépendamment dans le groupe constitué de : halogénoalkyle en C₁₋₈, -OH, halogène, -NO₂, -CN, alkyle en C₁₋₈, alcoxy en C₁₋₈, alcényle en C₂₋₈, alcynyle en C₂₋₈ et -N(R^{a})₂,
R⁴ est sélectionné dans le groupe constitué de : H, α-aminoacyle ; -S(O)₂N(R^{a})₂, - C(O)N(R^{a})₂ et -C(O)R^{b};
chaque R^{a} est sélectionné indépendamment dans le groupe constitué de H et alkyle en C₁₋₈, et
R^{b} représente un alkyle en C₁₋₈ ;
à condition que :
si Z représente -CH₂- en position para par rapport à -OR⁴, que les indices m et n valent 0 et que R⁴ représente H, alors au moins un R³ est sélectionné dans le groupe constitué de : halogénoalkyle en C₁₋₈, halogène, -NO₂, alkyle en C₂₋₈, alcoxy en C₁₋₈, alcényle en C₂₋₈, alcynyle en C₂₋₈ et -N(R^{a})₂,
si Z représente -CH₂- en position para par rapport à -OR⁴ et que le composé est un 2,3,4-trihydroxybenzamide, alors au moins un R² ou au moins un R³ est sélectionné dans le groupe constitué de : -OH, -NO₂, -CN, alcoxy en C₁₋₈, halogénoalkyle en C₁₋₈, alcényle en C₂₋₈, alcynyle en C₂₋₈ et -N(R^{a})₂,
si Z représente -CH₂- en position para par rapport à -OR⁴ et que le composé représente un 5-alkyl-6-hydroxybenzamide, alors au moins un R² ou au moins un R³ est sélectionné dans le groupe constitué de : -OH, -NO₂, -CN, alkyle en C₁₋₈, alcoxy en C₁₋₈, halogénoalkyle en C₁₋₈, alcényle en C₂₋₈, alcynyle en C₂₋₈ et -N(R^{a})₂,
si Z représente -C(O)- en position para par rapport à -OR⁴ et que le composé représente un 2-hydroxybenzamide, un 2,5-dialkyl-6-hydroxybenzamide ou un 5-alkyl-6-hydroxybenzamide, alors (i) au moins un R² ou au moins un R³ est sélectionné dans le groupe constitué de : -OH, alcoxy en C₁₋₈, halogénoalkyle en C₁₋₈, alcényle en C₂₋₈, alcynyle en C₂₋₈ et -N(R^{a})₂ ; et (ii) au moins un R³ est différent de -CF₃ ou de -NO₂ lorsque l'indice n vaut 1 et que R² représente 4-chloro ou 4-(*n-butyle*), et
si Z représente -S- en position para par rapport à -OR⁴ et que le composé représente un 2,5-dialkyl-6-hydroxybenzamide ou un 5-alkyl-6-hydroxybenzamide, alors au moins un R² ou au moins un R³ est sélectionné dans le groupe constitué de : -OH, alkyle en C₂₋₈, alcoxy en C₂₋₈, alcényle en C₂₋₈, alcynyle en C₂₋₈ et -N(R^{a})₂.

2. Composé selon la revendication 1, ou sel pharmaceutiquement acceptable de celui-ci, dans lequel :
(A) Z représente -CH₂-, -C(O)- ou -O-, et/ou
(B) l'indice m vaut 0, et/ou
(C) chaque R³ est sélectionné indépendamment dans le groupe constitué de : (i) halogénoalkyle en C₁₋₈, -OH, halogène, -NO₂, -CN, alkyle en C₁₋₈, alcoxy en C₁₋₈, alcényle en C₂₋₈ et alcynyle en C₂₋₈, ou (ii) halogénoalkyle en C₁₋₈, halogène et -NO₂.

3. Composé selon la revendication 1 ou 2, ou sel pharmaceutiquement acceptable de celui-ci, dans lequel l'indice p vaut 1 ou 2.

4. Composé selon la revendication 3, présentant une structure :
(A) selon la formule IIa : 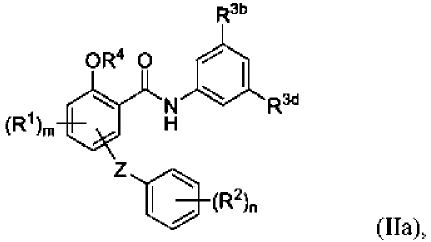 ou sel pharmaceutiquement acceptable de celui-ci, dans lequel éventuellement R^{3b} et R^{3d} (i) représentent un halogénoalkyle en C₁₋₈ ou (ii) sont sélectionnés indépendamment dans le groupe constitué de -CF₃ et -CCl₃, ou
(B) selon la formule IIb : 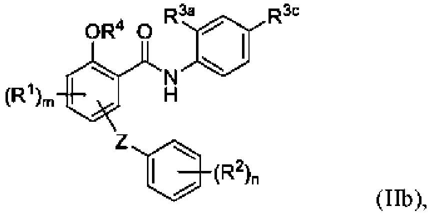 ou sel pharmaceutiquement acceptable de celui-ci, dans lequel éventuellement (i) R^{3a} et R^{3c} sont sélectionnés indépendamment dans le groupe constitué de : halogène et -NO₂, ou (ii) R^{3a} représente -Cl et R^{3c} représente -NO₂, ou
(C) selon la formule IIc : 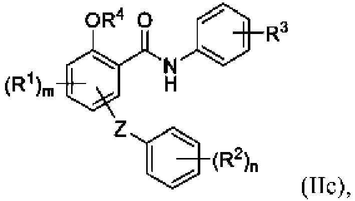 ou sel pharmaceutiquement acceptable de celui-ci, dans lequel éventuellement R³ (i) représente un halogénoalkyle en C₁₋₈ ou (ii) est sélectionné dans le groupe constitué de -CF₃ et -CCl₃.

5. Composé selon l'une quelconque des revendications 1 à 4, ou sel pharmaceutiquement acceptable de celui-ci, dans lequel l'indice n vaut 0.

6. Composé selon l'une quelconque des revendications 1 à 4, ou sel pharmaceutiquement acceptable de celui-ci, dans lequel l'indice n vaut 1, et R² est éventuellement sélectionné dans le groupe constitué de : alkyle en C₁₋₈, alcoxy en C₁₋₈ et halogénoalkyle en C₁₋₈.

7. Composé selon la revendication 6, présentant une structure :
(A) selon la formule IIIa : 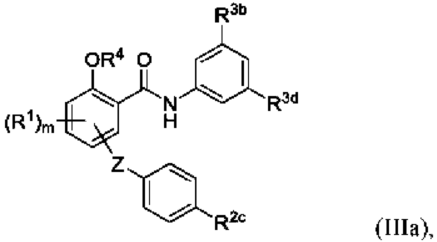 ou sel pharmaceutiquement acceptable de celui-ci, dans lequel éventuellement R^{3b} et R^{3d} (i) représentent un halogénoalkyle en C₁₋₈ ou (ii) sont sélectionnés indépendamment dans le groupe constitué de -CF₃ et -CCl₃, ou
(B) selon la formule IIIb : 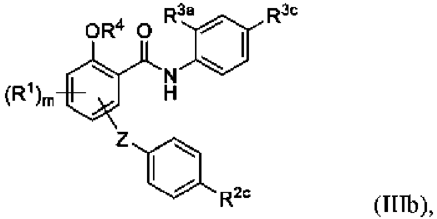 ou sel pharmaceutiquement acceptable de celui-ci, dans lequel éventuellement (i) R^{3a} et R^{3c} sont sélectionnés indépendamment dans le groupe constitué de : halogène et -NO₂, ou (ii) R^{3a} représente -Cl et R^{3c} représente -NO₂, ou
(C) selon la formule IIIc : 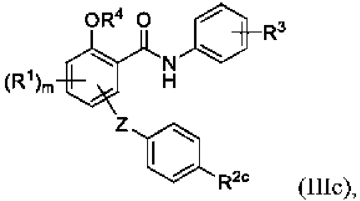 ou sel pharmaceutiquement acceptable de celui-ci, dans lequel éventuellement R³ (i) représente un halogénoalkyle en C₁₋₈ ou (ii) est sélectionné dans le groupe constitué de -CF₃ et -CCl₃.

8. Composé selon la revendication 7, ou sel pharmaceutiquement acceptable de celui-ci, dans lequel R^{2c} représente -OCH₃.

9. Composé selon l'une quelconque des revendications 1 à 8 ou sel pharmaceutiquement acceptable de celui-ci, dans lequel R⁴ (i) représente H ou α-aminoacyle, ou (ii) est sélectionné dans le groupe constitué de 1-valinyle et d-valinyle.

10. Composé selon la revendication 1, sélectionné dans le groupe constitué de : 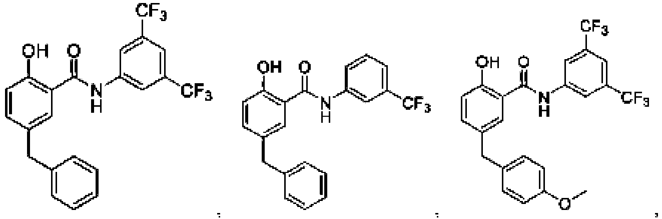 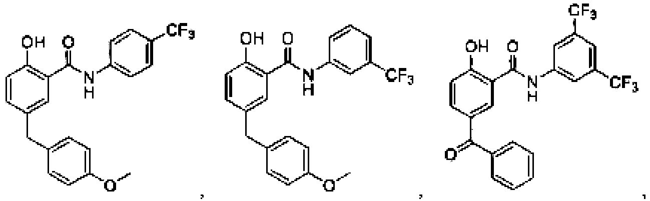 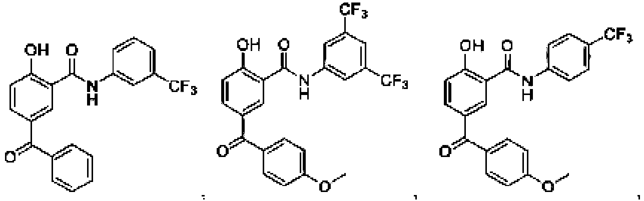 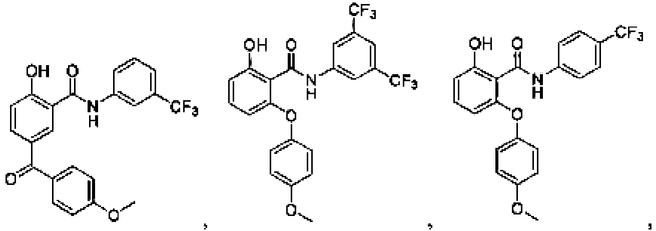 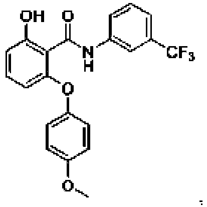 et sels pharmaceutiquement acceptables de ceux-ci.

11. Composé selon la revendication 1, ou sel pharmaceutiquement acceptable de celui-ci, dans lequel
(A) Z représente -CH₂- ; les indices m et n valent 0 ; et au moins un R³ est sélectionné dans le groupe constitué de : halogène, -NO₂, alcoxy en C₁₋₈, halogénoalkyle en C₁₋₈, alcényle en C₂₋₈, alcynyle en C₂₋₈ et -N(R^{a})₂, ou
(B) Z représente -C(O)- en position para par rapport à -OR⁴ ; le composé représente un 2-hydroxybenzamide, un 2,5-dialkyl-6-hydroxybenzamide ou un 5-alkyl-6-hydroxybenzamide ; et au moins un R² ou au moins un R³ est sélectionné dans le groupe constitué de : -OH, alcoxy en C₁₋₈, alcényle en C₂₋₈, alcynyle en C₂₋₈ et -N(R^{a})₂, ou
(C) Z représente -S- en position para par rapport à -OR⁴ ; le composé représente un 2,5-dialkyl-6-hydroxybenzamide ou un 5-alkyl-6-hydroxybenzamide ; et au moins un R² ou au moins un R³ est sélectionné dans le groupe constitué de : -OH, alkyle en C₂₋₈, alcényle en C₂₋₈, alcynyle et -N(R^{a})₂.

12. Composition pharmaceutique comprenant un composé selon l'une quelconque des revendications 1 à 11, ou sel pharmaceutiquement acceptable de celui-ci, et un excipient pharmaceutiquement acceptable.

13. Composé selon l'une quelconque des revendications 1 à 11, ou composition pharmaceutique selon la revendication 12, pour utilisation en tant que médicament.

14. Composé selon l'une quelconque des revendications 1 à 11, ou composition pharmaceutique selon la revendication 12, pour utilisation dans une méthode de traitement d'une maladie ou d'une affection médiée par les hormones, la méthode comprenant l'administration à un sujet qui en a besoin d'une quantité thérapeutiquement efficace du composé ou de la composition pharmaceutique, traitant ainsi la maladie ou l'affection médiée par les hormones, la maladie médiée par les hormones étant éventuellement un cancer, le cancer étant éventuellement un cancer de la prostate résistant à la castration.

15. Composé ou composition pharmaceutique pour utilisation selon la revendication 14, la méthode comprenant en outre l'administration d'un agent antiandrogène au sujet, l'agent antiandrogène étant éventuellement sélectionné dans le groupe constitué de : enzalutamide, apalutamide, darolutamide, abiratérone, les sels pharmaceutiquement acceptables de ceux-ci, et les combinaisons de ceux-ci.
